# EUROPEAN PATENT SPECIFICATION

(11) **EP 4 031 236 B1**
(45) Date of publication and mention of the grant of the patent: **25.11.2026**
(21) Application number: 20865710.6
(22) Date of filing: 16.09.2020
(51) Int. Cl.: A61N 1/05, A61B 18/14, A61B 90/00, A61N 1/36, A61N 1/372, A61N 1/375, A61B 17/32, A61B 17/29

(54) **NEURO STIMULATOR ARRANGEMENTS**
NEURO-STIMULATORANORDNUNGEN
AGENCEMENTS DE NEURO-STIMULATEURS

(30) Priority: 17.09.2019 US 201962901565 P
(43) Date of publication of application: 27.07.2022
(73) Proprietor: Stryker Corporation, Portage, MI 49002 (US)
(72) Inventor: CARR, Jacob, J., Indianapolis, IN 46220 (US); JARIAL, Inder, Indianapolis, IN 46240 (US)
(74) Representative: Schott, Jakob Valentin
(86) International application number: PCT/US2020/051054
(87) International publication number: WO 2021/055462

(56) References cited:
- WO-A1-2014/074237
- US-A- 5 925 041
- US-A1- 2006 004 419
- US-A1- 2007 016 185
- US-A1- 2007 100 336
- US-A1- 2007 239 187
- US-A1- 2012 316 590
- US-A1- 2017 042 528
- US-A1- 2017 071 667
- US-A1- 2017 196 557
- US-A1- 2019 159 855
- US-B1- 6 298 256
- US-B1- 9 028 522
- US-B2- 10 080 578

## Description

### TECHNICAL FIELD

The present disclosure relates generally to neuro stimulator arrangements that may be used with tissue removal devices, in particular, tissue removal devices that are suited for neurosurgical and spinal surgical procedures.

### BACKGROUND

Various abnormalities of the neurological system, such as brain and spinal tumors, cysts, lesions, or neural hematomas, can cause severe health risks to patients afflicted by them, including deterioration in motor skills, nausea or vomiting, memory or communication problems, behavioral changes, headaches, or seizures. In certain cases, resection of abnormal tissue masses is required. However, given the complexity and importance of the neurological system, such neurosurgical procedures are extremely delicate and must be executed with great precision and care. Many known tissue removal devices, including, but not limited to tissue cutting devices and aspirators, suffer from an inability to quickly and cleanly sever neurological tissue samples without causing "traction" or pull on the surrounding tissue. In addition, many known devices are not configured to both "debulk" large structures and to finely shave smaller, more delicate structures and lack the flexibility needed in many procedures. Furthermore, many neurological procedures impose significant space limitations on the surgeon, and the tissue removal device needs to be manipulable by the surgeon with one hand in relatively small spaces. Many known devices either emulsify the resected tissue, macerate the resected tissue, or thermally damage the tissue rendering it unsuitable for subsequent analysis (e.g., pathologic and/or histologic analysis) which is necessary for the determination of the most effective post resection treatment therapies. Thus, a need has arisen for a tissue removal device that addresses the foregoing issues.

While advances in neurosurgery have allowed greater access to abnormalities while minimizing further damage to promote treatment, great care still needs to be exercised in accessing the subcortical space within the brain. To avoid potential damage to the brain during treatment, a known technique developed by Wilder Penfield and Herbert Jasper provided for stimulation of the brain with electrical probes while patients were conscious to observe the responses to provide targeted therapy. This technique allowed for creating maps of the sensory and motor cortices of the brain to show their connections to the various limbs and organs of the body. This technique, now known as Electrocorticography (EcoG), intracranial electroencephalography (EEG), monopolar cortex stimulation (MCS) are types of electrophysiological monitoring that use electrodes placed directly on an exposed surface of the brain to record activity from the cerebral cortex rather than conventional EEG, where electrode monitors this activity from outside the skull. EcoG allows for direct electrical stimulation of the brain, identifying critical regions of the cortex to be avoided during surgery, including electrocortical mapping and continuous intraoperative neurophysiological monitoring.

Electrical stimulation is a validated intraoperative technique for identifying the motor fibers in deep white matter tracts. While this process provides for monitoring of critical brain activity, in practice, the procedure uses intermittent subcortical mapping with a separate handheld probe to localize critical fiber tracts and to reduce the risk of motor deficits. However, during tissue removal or manipulation procedures, the surgeon does not exactly know where such critical structures are located, unless the procedure is stopped and the separate mapping probe is reinserted to explore the tissue cavity point by point. What is needed is a device that provides for EcoG monitoring while a surgical procedure is being conducted, without the need to remove a tissue removal device from an area of interest.

US 2007/0100336 A1 discloses a resecting instrument including an outer tube forming a cutting window at which a cutting tip of an inner member is located. An electrically non-conductive material covers a region of the outer tube, and wiring is connected to an exposed surface of the outer tube.

US 2007/0239187 A1 discloses a guard for use with a surgical cutting system. The guard includes a housing and wiring. The housing defines a longitudinal passageway, and is configured for releasable attachment to an instrument handpiece. The wiring is coupled to the housing and includes an electrically conductive wire and an insulative material. The first end of the wire establishes an electrical connection with a cutting tool shank upon placement within the passageway.

### SUMMARY

The invention is defined by claim 1. Further embodiments of the invention are defined by the dependent claims.

Different arrangements for neuro stimulator arrangements are disclosed herein. In one exemplary arrangement, a neuro stimulator adapter for a tissue removal device is disclosed. The neuro stimulator adapter includes an engagement sleeve and a hub. The hub further has a contact assembly disposed therein, the contact assembly configured to be connected to an electrical source. The engagement sleeve is fixedly connected to a portion of the hub. In operation, a cannula of the tissue removal device is inserted within the engagement sleeve and the hub is connected to a portion of the tissue removal device such that electrical stimulation may be provided through the cannula, thereby permitting a single device to be used as a both a tissue removal device and a neuro stimulator to assist in avoiding critical structures as part of a surgical procedure.

In another exemplary arrangement, a tissue removal device that has a neuro stimulator arrangement is disclosed. The tissue removal device comprises a cannula, a handpiece, an engagement sleeve, and hub. A proximal end of the cannula is connected to the handpiece. The engagement sleeve is fixedly connected to a portion of hub. The hub has a contact assembly disposed therein. The contact assembly configured to be connected to an electrical source. The hub is selectively attached to a portion of the handpiece in an operation mode such that the cannula is received with in the engagement sleeve, with a distal end of the cannula protruding distally from the engagement sleeve. A proximal section of the cannula is in electrical contact with contact assembly so as to provide electrical stimulation through the cannula.

In a further exemplary arrangement, a neuro stimulator adapter for a tissue removal device is disclosed. In one exemplary arrangement, the neuro stimulator adapter comprises a communication cannula; a communicating member; and a connecting hub. The communication cannula is defined by a distal end and a proximal end and the proximal end is fixedly secured to the hub. The communication cannula further includes an inner insulating layer, a communicating layer and an outer layer. The communicating layer further includes an extended portion that extends distally from the inner insulating layer and the outer layer and the communicating member extends from the communicating layer. In operation, the communication cannula is slid over a cannula of the tissue cutting device. In this manner, the communicating member is disposed adjacent a tissue cutting opening of the cannula, thereby allowing for neuro stimulation and monitoring of the tissue adjacent to the tissue cutting device.

An embodiment of a neuro stimulator adapter assembly for use with a tissue removal device is disclosed. The embodiment of the neuro stimulator adapter assembly comprises at least one connector element, a contact element and a communicating wire. The connector element may comprise a main body section and one or more clip members. The main body section has the contact element hingedly attached thereto. The communicating wire is operatively connected to the main body section. In operation, the clip member is secured to a portion of a handpiece of a tissue removal device such that an inner cannula of the tissue removal device is in electrical contact with the contact. In this manner, electrical stimulation may be provided through the inner cannula such that the inner cannula can stimulate and allow monitoring of tissue coming into contact with the inner cannula before a surgical intervention operation commences.

Another exemplary arrangement of a neuro stimulator arrangement is disclosed that includes a connecting hub, an engagement sleeve and a communicating member. In this arrangement, the communicating member may be constructed as a neurostimulation wire or probe. In one exemplary arrangement, the communicating member is attached to an outside surface of the engagement sleeve. In one exemplary arrangement, the communicating member may be co-extruded with the engagement sleeve. In one exemplary arrangement, the communicating member may include an insulation layer and a conductive area, with only the conductive area being exposed at a distal end of the communicating member, adjacent a distal end of the engagement sleeve. In operation, the engagement sleeve is slid over a cannula of the tissue removal device. In this manner, the communicating member is disposed adjacent a tissue opening of the cannula, thereby allowing for neuro stimulation and monitoring of the tissue adjacent to the tissue removal device during use of the tissue removal device.

### BRIEF DESCRIPTION OF THE DRAWINGS

Exemplary embodiments of the present disclosure will now be described in greater detail with reference to the attached figures, in which:
FIG. 1 is a perspective view of an exemplary tissue removal device in accordance with a first embodiment;
FIG. 2 is a cross-sectional view of the tissue removal device of FIG. 1 depicting an inner cannula in a first relative position with respect to an outer cannula in which the inner cannula's distal end is located proximally of the outer cannula's distal end;
FIG. 3 is a broken side elevation view of the outer cannula of the tissue removal device of FIG. 1;
FIG. 4 is a broken side elevation view of the inner cannula of the tissue removal device of FIG. 1;

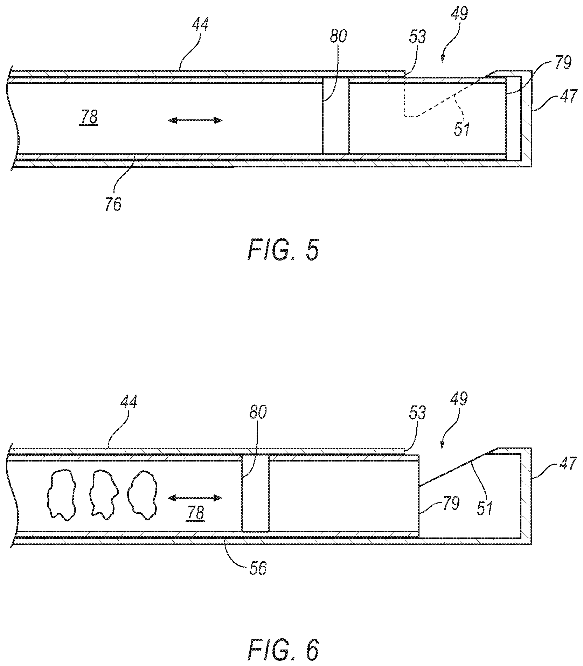

FIG. 5 is a partial cross-sectional view of a distal region of the outer cannula and the inner cannula of the tissue removal device of FIG. 1, depicting the inner cannula in a second relative position with respect to the outer cannula;
FIG. 6 is a partial cross-sectional view of a distal region of the outer cannula and the inner cannula of the tissue removal device of FIG. 1, depicting the inner cannula in the first relative position with respect to the outer cannula;
FIG. 7 is an exploded assembly view of the tissue removal device of FIG. 1;
FIG. 8 is a partial perspective view of a portion of the tissue removal device of FIG. 1 with an upper shell of an outer sleeve upper housing removed to show a dial for rotating the outer cannula;
FIG. 9 is a side elevation view of an inner and outer cannula assembly of the tissue removal device of FIG. 1;
FIG. 10A is a perspective view of an neuro stimulator adapter and a portion of the outer sleeve upper housing;
FIG. 10B is a front elevational view of the neuro stimulator adapter of FIG. 10A;
FIG. 10C is a side elevational view of the neuro stimulator adapter positioned within the outer sleeve upper housing;
FIG. 10D is a top perspective view of the neuro stimulator adapter positioned within the outer sleeve upper housing;
FIG. 11A is a partially exploded view of the neuro stimulator adapter mounted to the outer sleeve upper housing and the inner and outer cannula assembly;
FIG. 11B is an assembled view of the inner and outer cannula assembly positioned within the outer sleeve upper housing;
FIG. 12A is a cross-sectional view of the assembled tissue removal device with the electro-simulator adapter mounted to the upper housing;
FIG. 12B is an enlarged view of the area 12B taken from FIG. 12A;
FIG. 12C is an annotated cross-sectional view of the assembled tissue removal device with the neuro stimulator adapter mounted to the upper housing and showing the electrical pathway created by the neuro stimulator adapter when in an activated state;
FIG. 13A is an exploded perspective view of an alternative arrangement of a neuro stimulator adapter and the tissue removal device of FIG. I;
FIG. 13B is a perspective view of the neuro stimulation adapter of FIG. 13A mounted to the tissue removal device;
FIG. 13C is an enlarged view of area 13C of FIG. 13B, illustrating a connection hub of the neuro stimulation adapter of FIG. 13A secured to the tissue removal device;
FIG. 14A is a perspective view of the neuro stimulation adapter of FIG. 13A;
FIG. 14B is an enlarged view of area 14B of FIG. 14A, illustrating a distal end of the neuro stimulation adapter;
FIG. 14C is a cross-sectional view of an insulated lumen of the neuro stimulation adapter of FIG. 13A, taken along lines 14C-14C in FIG. 14A;
FIG. 15A is a perspective view of a distal end of the tissue removal device with the neuro stimulation adapter secured thereto;
FIG. 15B is an enlarged view of area 15B of FIG. 15A, illustrating the distal end of the tissue removal device;
FIG. 16A is a partially exploded perspective view of an alternative arrangement of a neuro stimulator adapter and the tissue removal device of FIG. 1;
FIG. 16B is a perspective view of the neuro stimulation adapter of FIG. 16A mounted to the tissue removal device;
FIG. 16C is an enlarged view of area 16C of FIG. 16B, illustrating a connection hub of the neuro stimulation adapter of FIG. 16A secured to the tissue removal device;
FIG. 17A is a perspective view of the neuro stimulation adapter of FIG. 16A;
FIG. 17B is a cross-sectional view of the dual lumens of the neuro stimulation adapter of FIG. 16A, taken along lines 17B-17B in FIG. 17A;
FIG. 17C is an enlarged view of area 17C of FIG. 17A, illustrating a distal end of the neuro stimulation adapter;
FIG. 18 is a perspective view of a distal end of the tissue removal device with the neuro stimulation adapter secured thereto;
FIGS. 19A-19C illustrate alternative arrangements of a communication tip;
FIG. 20A is a partial side elevational view of an alternative arrangement of an adapter sleeve mounted to a tissue removal device;
FIG. 20B is an enlarged side elevational view of a distal end of a delivery sleeve mounted to the tissue removal device, with a neurostimulation probe in a first position;
FIG. 20C is an enlarged side elevational view of the distal end of the delivery sleeve mounted to the tissue removal device, with a neuro stimulation probe in a second position;
FIG. 21A is a perspective view of a further alternative arrangement of a neuro stimulation adapter secured to a tissue removal device;
FIG. 21B is a cross-sectional view of the neuro stimulation adapter of FIG. 21A;
FIG. 21C is an enlarged view of area 21C in FIG. 21B;
FIG. 22A is a rear perspective view of a delivery sleeve hub;
FIG. 22B is a transparent side elevational view of the delivery sleeve hub of FIG. 22A;
FIG. 22C is a transparent rear elevational view of the delivery sleeve hub of FIG. 22A;
FIG. 23A is a rear perspective view of the delivery sleeve hub of FIG. 22A mounted to upper housing of a tissue removal device;
FIG. 23B is a top perspective view of a portion of the delivery sleeve hub of FIG. 22A mounted to the tissue removal device;
FIG. 24A is an annotated cross-sectional view of the delivery sleeve hub of FIG. 22A mounted to the tissue removal device;
FIG. 24B is an enlarged annotated cross-sectional view of area 24C taken from FIG. 24A;
FIG. 25A is a perspective view of a further alternative arrangement of a neuro stimulation arrangement secured to a tissue removal device; and
FIG. 25B is a cross-sectional view of the neuro stimulation arrangement of FIG. 25A.

### DETAILED DESCRIPTION

Referring now to the discussion that follows and also to the drawings, illustrative approaches to the disclosed assemblies and methods are shown in detail. Although the drawings represent some possible approaches, the drawings are not necessarily to scale and certain features may be exaggerated, removed, or partially sectioned to better illustrate and explain the present disclosure. Further, the descriptions set forth herein are not intended to be exhaustive or otherwise limit or restrict the claims to the precise forms and configurations shown in the drawings and disclosed in the following detailed description.

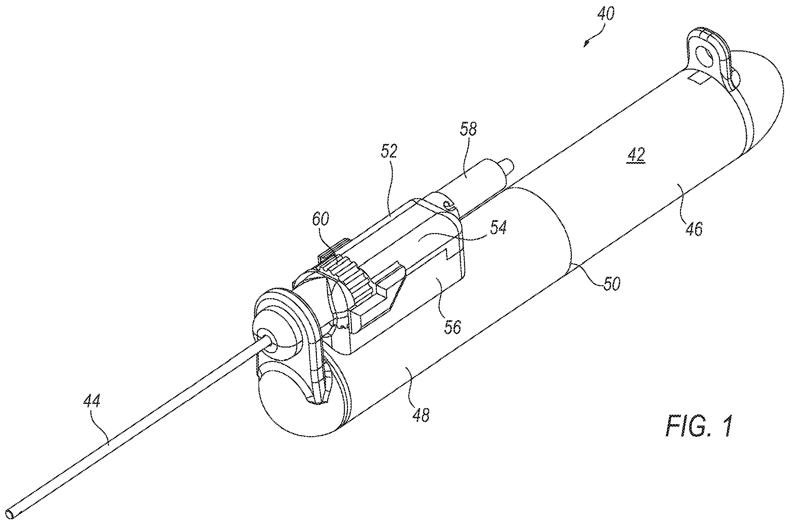

Described herein are tissue removal devices that are suited for neurosurgical applications such as the removal of spine and brain tissue. The components disclosed herein provide surgeons with an enhanced ability to minimize trauma to the patient, while providing efficient improved minimally invasive surgical techniques, such as, for example, during intracranial surgical techniques. The components disclosed herein may further be used for application of targeted and effective treatment regimens. Referring to FIG. 1, an exemplar of a tissue removal device 40 is shown. However, it is understood that the concepts disclosed herein may be used with other tissue removal devices, including aspirating devices and treatment devices. With respect to the identified exemplar tissue removal device 40 may be configured similarly to the tissue removal device shown and described in commonly owned U.S. Patent No. 9,387,010. However, it is understood that other tissue removal devices may be employed.

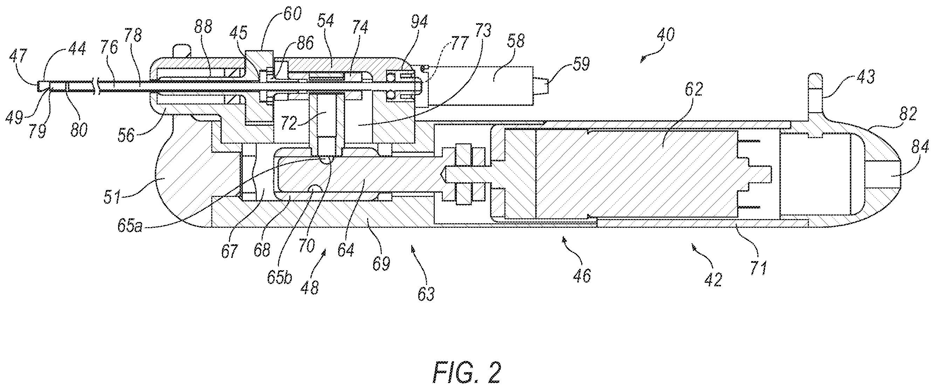

In one exemplary arrangement, tissue removal device 40 includes a handpiece 42 and an outer cannula 44. Handpiece 42 includes a lower housing 50 which comprises a proximal section 46 and distal section 48. Lower housing 50 comprises a proximal-most housing portion 82 (FIG. 2) that is connected to a motor housing 71, and a cam housing 69 that is connected to motor housing 71. A front housing section 51 is connected to cam housing 69. Upper housing 52 is also provided. A tissue collector 58 may be operatively connected to upper housing 52, either directly or indirectly. A rotation dial 60 for selectively rotating the outer cannula 44 with respect to handpiece 50 is also mounted to upper housing 52.

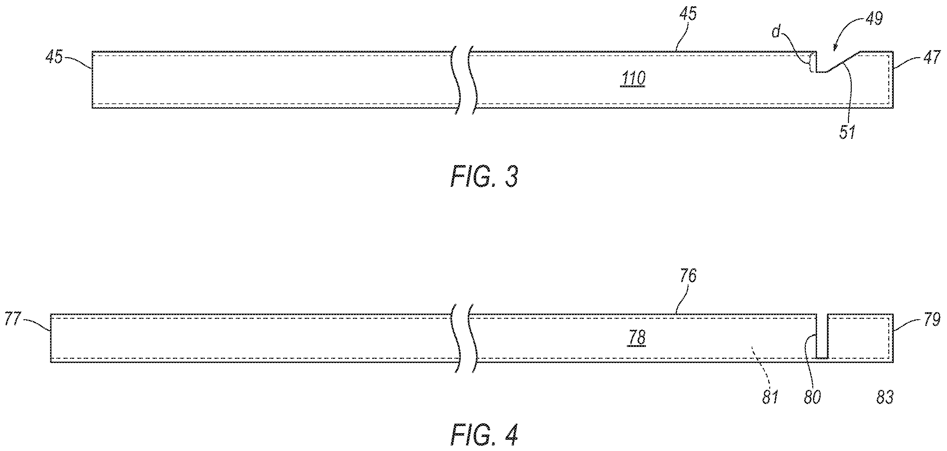

As best seen in FIGS. 2-3 and 9, outer cannula 44 includes an open proximal end 45, a closed distal end 47, and a distal opening 49 proximate distal end 47. Tissue cutting device 40 further comprises an inner cannula 76 which is partially disposed in an outer cannula lumen 110. Inner cannula 76 is configured to reciprocate within outer cannula lumen 110 and to cut tissue samples entering outer cannula 44 via outer cannula distal opening 49. Inner cannula 76 reciprocates between a proximal position and a distal position. Referring to FIG. 4, inner cannula 76 includes an open proximal end 77 and an open distal end 79. Distal end 79 is configured to cut tissue, and in certain exemplary arrangements is configured for cutting neurological system tissues such as those from the brain or spine. In one exemplary arrangement, a hinge 80 (described in further detail below), may be provided to facilitate cutting.

Outer cannula 44 is not translatable, and its position with respect to handpiece 42 along the direction of the longitudinal axis of handpiece 42 remains fixed. Motor 62 is disposed in proximal lower housing section 46 of handpiece 42 and is operably connected to inner cannula 76 to drive the reciprocation of inner cannula 76 within outer cannula 110. Motor 62 may be a reciprocating or rotary motor.

Motor 62 is housed in motor housing 71, which defines a portion of lower housing proximal section 46. Motor 62 is connected to an inner cannula drive assembly 63 which is used to convert the rotational motion of motor 62 into the translational motion of inner cannula 76. At its proximal end, motor housing 71 is connected to proximal-most housing portion 82, which includes a power cable port 84 and a hose connector 43, which in the exemplary embodiment of FIG. 2 is an eyelet. Hose connector 43 provides a means of securely retaining a vacuum system hose to handpiece 42, thereby allowing vacuum pressure to be supplied to tissue collector 58.

Inner cannula driver assembly 63 comprises a cam 64, a cam follower 68, a cam transfer 72, and a cannula transfer 74. Cam 64 is a generally cylindrical structure and has a groove or channel 65 defined in the surface of cam 64. In one exemplary embodiment, groove 65 is continuous and circumscribes the perimeter of cam 64 but is not oriented perpendicularly to the longitudinal axis of cam 64, i.e., groove 65 is angled with respect to the cam axis. Opposing points on groove 65 such as points *65a* and *65b* define pairs of "apexes" that are spaced apart along the longitudinal axis of the cam, i.e., the groove extends along a portion of the length of the cam. Cam 64 also includes a proximal opening (not shown) for receiving a motor shaft and a proximal recess (not shown) into which a shaft may be snugly received.

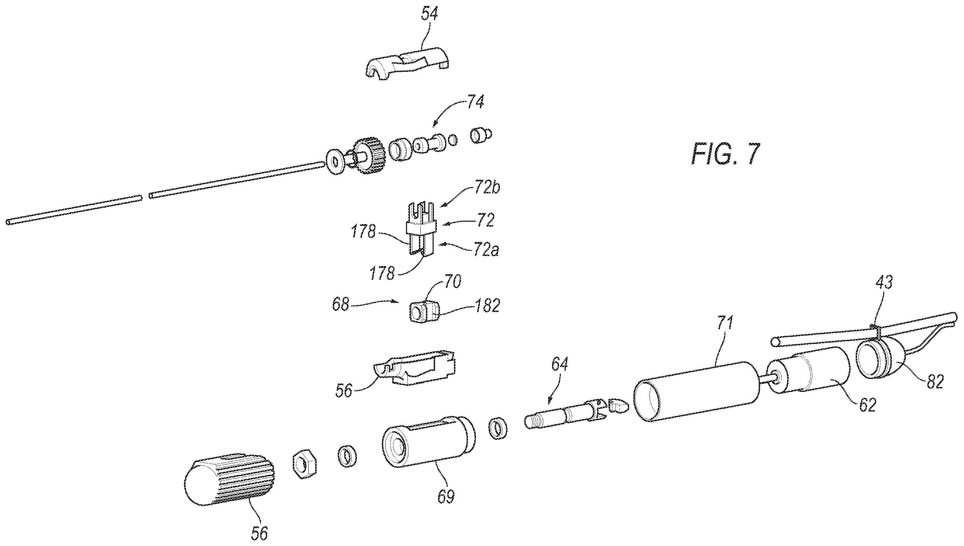

In one embodiment, cam follower 68 (seen in FIG. 7) is a generally rectangular block shaped structure with a hollow interior in which cam 64 is partially disposed. Cam follower 68 also includes a hole 70 in its upper face in which a ball bearing (not shown) is seated. The ball bearing rides in cam groove 65 and engages cam transfer 72. As a result, when cam 64 rotates, cam follower 68 translates along the length of handpiece 42. Cam follower 68 also includes lateral slots 182 that cooperatively engage corresponding members 178 from cam transfer 72.

Cam follower 68 is disposed within a cam chamber 67 formed in cam housing 69. Cam 64 is partially disposed in cam chamber 67 and extends proximally therefrom to engage motor 62. Cam 64 does not reciprocate within cam chamber 67 and instead rotates about its own longitudinal axis. Cam follower 68 reciprocates within cam chamber 67 along the direction of the length of handpiece 42. Cam follower 68 is open at its proximal end to receive cam 64.

Cam transfer 72 (best seen in FIG. 7) extends from cam chamber 67 into a cam transfer chamber 73 formed in upper housing 52. Cam transfer 72 comprises a proximal end *72a* that is attachable to cam follower 68 and a distal end *72b* that is attachable to inner cannula 76 via cannula transfer 74.

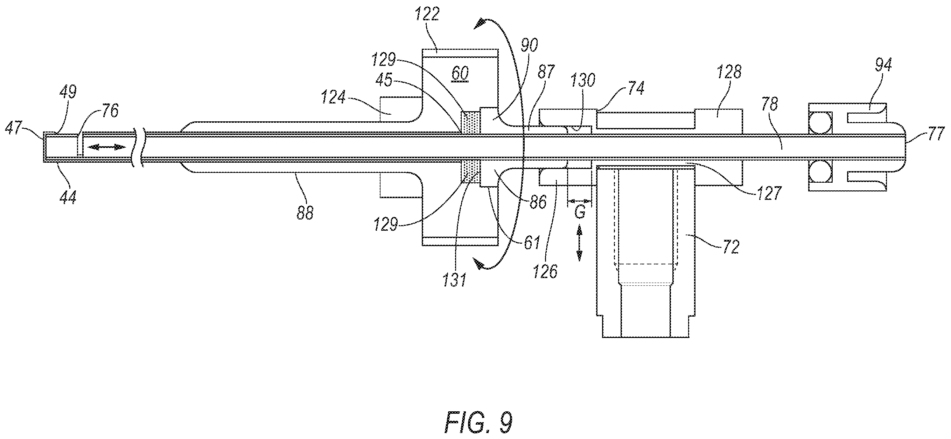

As best seen in FIG. 9, cannula transfer 74 comprises a sleeve disposed about inner cannula 76. Cannula transfer 74 comprises a proximal end 128, middle section 127, and distal end 126. Upwardly extending members of cam transfer 72 define fork-shaped structures that receive and cradle middle section 127 of cannula transfer 74. Distal end 126 and proximal end 128 of cannula transfer 74 are disposed outwardly of upwardly extending members and are shaped to prevent relative translation between cam transfer 72 and cannula transfer 74. In the depicted embodiments, distal end 126 and proximal end 128 of cannula transfer 74 are enlarged relative to middle section 127 to abut the upwardly extending members, thereby preventing relative translation between cam transfer 72 and cannula transfer 74. As a result, when cam transfer 72 reciprocates along the length of handpiece 42, cannula transfer 74 reciprocates as well. Because it is affixed to inner cannula 76, when cannula transfer 74 reciprocates, it causes inner cannula 76 to reciprocate within outer cannula 44.

Cam transfer 72 may be connected to cam follower 68 by mechanical means, adhesive means or other known connection means. In one exemplary embodiment, downwardly extending members 178 mechanically clip onto and removably engage cam follower 68. In another embodiment, cam transfer 72 is adhesively affixed to cam follower 68. In yet another embodiment, both mechanical and adhesive connections are used. The ball bearing (not shown) disposed in cam follower hole 70 traverses cam groove 65 as cam 64 rotates, causing cam follower 72 to reciprocate from the proximal position of FIG. 2 to the distal position of FIG. 3. As a result, cam transfer 72, cannula transfer 74 and inner cannula 76 translate between their respective proximal positions and their respective distal positions when motor 62 and cam 64 rotate. In certain examples (not separately shown), motor 62 may be connected to a cam follower, and the cam follower may be connected to a cam which is in turn operatively connected to the inner cannula. In accordance with these examples, when the motor rotates, the cam follower rotates and causes the cam to reciprocate, thereby causing the inner cannula to reciprocate.

Outer cannula 44 includes an opening 49 for receiving tissue into outer cannula lumen 110. Opening 49 may be defined by a cutting edge 51 that is configured to sever tissue and a non-cutting edge 53 that is not configured to sever tissue. Inner cannula distal end 79 is preferably configured to cut tissue. As tissue is received in outer cannula opening 49, it is compressed between inner cannula distal end 79 and outer cannula cutting edge 51, causing the received tissue to be severed from the surrounding tissue.

Inner cannula 76 may include a hinge 80. Hinge 80 is located between inner cannula body section 81 which is located on the proximal side of hinge 80 and inner cannula cutting section 83 which is located on the distal side of hinge 80. Hinge 80 allows cutting section 83 to pivot about hinge 80 as inner cannula 76 reciprocates within outer cannula 44. As inner cannula 76 translates in the distal direction, it contacts tissue received in outer cannula opening 49 and encounters progressively increasing resistance from the tissue as the tissue is urged in the distal direction. As the resisting force of the tissue increases, cutting section 83 pivots progressively more until a zero annular clearance is obtained between inner cannula distal end 79 and outer cannula opening 49. The received tissue is severed and aspirated in the proximal direction along inner cannula lumen 110 and received in tissue collector 58. Thus, inner cannula lumen 110 provides an aspiration path from the inner cannula distal end 79 to the inner cannula proximal end 77.

Tissue cutting device 40 aspirates tissue samples received in inner cannula lumen 78 to cause the tissue samples to move in the proximal direction along the length of the inner cannula 76. In some exemplary embodiments, device 40 preferably includes a tissue collector 58 into which aspirated tissue samples are deposited during a tissue removal procedure. Tissue collector 58 may be located remotely from handpiece 42 and outside the sterile field during a tissue removal operation or may be removably connected to handpiece 40. Referring to FIG. 2, in one exemplary arrangement, a vacuum hose fitting 59 is formed on the proximal end of tissue collector 58 and is in fluid communication with the interior of tissue collector 58 and with a vacuum generator, as will be discussed below.

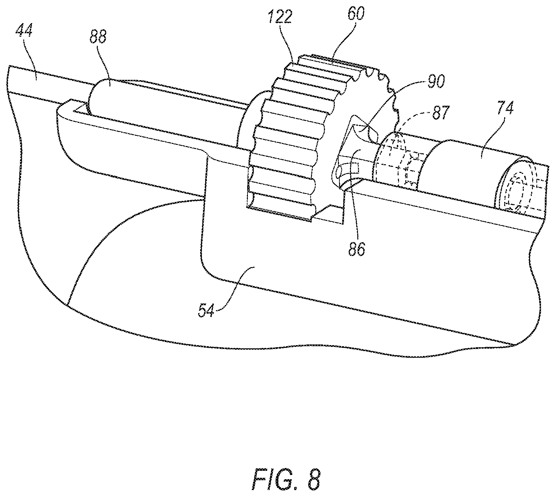

Referring to FIGS. 2, and 7-9, upper shell 54 and lower shell 56 of upper housing 52 cooperatively define a cavity into which a seal holder 94 (best seen in FIG. 9) is partially disposed. Seal holder 94 also includes a central lumen through which inner cannula 76 is slidably disposed. As best seen in FIG. 2, inner cannula proximal end 77 preferably remains within seal holder 94 as inner cannula 76 reciprocates during operation of tissue removal device 40. However, proximal end 77 moves within seal holder 94 as inner cannula 76 reciprocates.

When device 40 is used to cut, aspirate or otherwise manipulate tissue, outer cannula opening 49 must be aligned with the target tissue of interest to receive it. In an exemplary embodiment, device 40 includes a selectively rotatable outer cannula 44. As best seen in FIG. 7-9, rotation dial 60 is provided and is rotatably seated in a cavity defined by upper shell 54 and lower shell 56 of upper housing 52. Rotation dial 60 is configured such that when it is rotated, it causes outer cannula 44 to rotate about its longitudinal axis. Rotation dial 60 is preferably connected to an outer cannula connector portion 88. In the embodiment of FIGS. 7-9, outer cannula connector portion 88 is a sleeve that is integrally formed with rotation dial 60 and which is fixedly secured to outer cannula 44 such as by an adhesive or other known connection means.

To ensure the correct operation of hinge 80 of inner cannula 76, the circumferential alignment of hinge 80 and outer cannula opening 49 should be maintained. Thus, rotation dial 60 is preferably connected to inner cannula 76 such that when rotation dial 60 is rotated, both outer cannula 47 and inner cannula 76 rotate in a fixed angular orientation with respect to one another by an amount that directly corresponds to the amount by which rotation dial 60 is rotated. Rotation dial 60 may be directly connected to inner cannula 76 or may use an intervening connecting device. However, rotation dial 60 should be configured to allow inner cannula 76 to reciprocate with respect to rotation dial 60. As best seen in FIG. 9, rotation dial inner cannula connector 86 may be provided to connect rotation dial 60 to inner cannula 76. Rotation dial inner cannula connector 86 comprises a proximal sleeve 87 disposed about inner cannula 76 and a distal, radially extending annular flange 90 with an outer diameter greater than that of the sleeve 87. Sleeve 87 is slidably received within the annular cavity 130 at the distal end 126 of the cannula transfer 74 and keyed to the inner surface of cannula transfer 74 at annular cavity 130 such that sleeve 87 can reciprocate with respect to cannula transfer 74 while causing cannula transfer 74 to rotate with sleeve 87 when rotation dial 60 is rotated. When inner cannula 76 is reciprocated, cannula transfer distal end 126 reciprocates with respect to sleeve 87, thereby variably adjusting gap "G" defined within annular cavity 130 (FIG. 9). Alternatively, cannula transfer distal end 126 may be slidably received in an annular cavity formed in sleeve 87 and may be keyed to the inner surface of the annular cavity so that cannula transfer may reciprocate with respect to sleeve 87 while still rotating with sleeve 87 when dial 60 is rotates.

As best seen in FIG. 9, rotation dial 60 includes a first annular cavity 61 that is sized to receive and engage flange 90 in a close fitting relationship. Rotation dial 60 may be press fit to flange 90. In addition, adhesive connections or mechanical connections may be used. Because rotation dial 60 is directly or indirectly connected to both outer cannula 44 and inner cannula 76, both cannulae rotate in direct correspondence to the rotation of rotation dial 60, thereby allowing the user to adjust the orientation of outer cannula opening 49 and inner cannula hinge 80 in a circumferential direction with respect to handpiece 42. As a result, surgeons need not rotate the entire tissue removal device 40 to obtain the desired angular orientation.

Rotation dial 60, outer cannula 44, and inner cannula 76 are preferably configured for 360° rotation. In addition, tactile indicators are preferably provided on rotation dial 60 to allow a user to reliably determine the extent to which dial 60 has been rotated from a given starting point. The tactile indication may comprise surface features defined on or in the exterior surface of rotation dial 60.

As mentioned previously, vacuum (sub-atmospheric pressure) is applied to tissue collector 58 to aspirate severed tissue samples through inner cannula 76 in the proximal direction. A seal 129 is preferably provided to prevent air artifacts, fluid (water, saline, blood, etc.) flow, and tissue sample flow in the annular clearance between inner cannula 76 and outer cannula 44. The seal 129 is preferably disposed adjacent the proximal end of the annular clearance between inner cannula 76 and outer cannula 44, i.e., proximally adjacent to outer cannula proximal end 45.

In the embodiment of FIG. 9, rotation dial 60 and sleeve 87 act as a seal housing and include a seal cavity 131 which is an annular cavity disposed immediately adjacent to and distal of first annular cavity 61. Seal cavity 131 is sized to accept seal 129 therein. The seal 129 may be a conventional seal such as a solid, flexible and/or elastomeric o-ring or may be a thixotropic material that is a semi-solid. It is further preferred that seal 129 fill the entirety of seal cavity 131 to ensure that cavity 131 is substantially leak free.

In one configuration, device 40 is connected to a vacuum source and configured for variable aspiration, i.e., configured to supply variable levels of vacuum to inner cannula lumen 78.

The system further includes an electrical controller (not shown) which receives and provides signals to the various components to control or monitor their operations. The controller provides control signals to device 40 via a motor drive control line to activate or deactivate motor 62. An aspiration valve control line extends from the controller to a controllable valve (not shown) which provides pressure to a vacuum generator (not shown). Signals to the controllable valve through the aspiration valve control line are used to control the amount of vacuum applied to tissue collector 58.

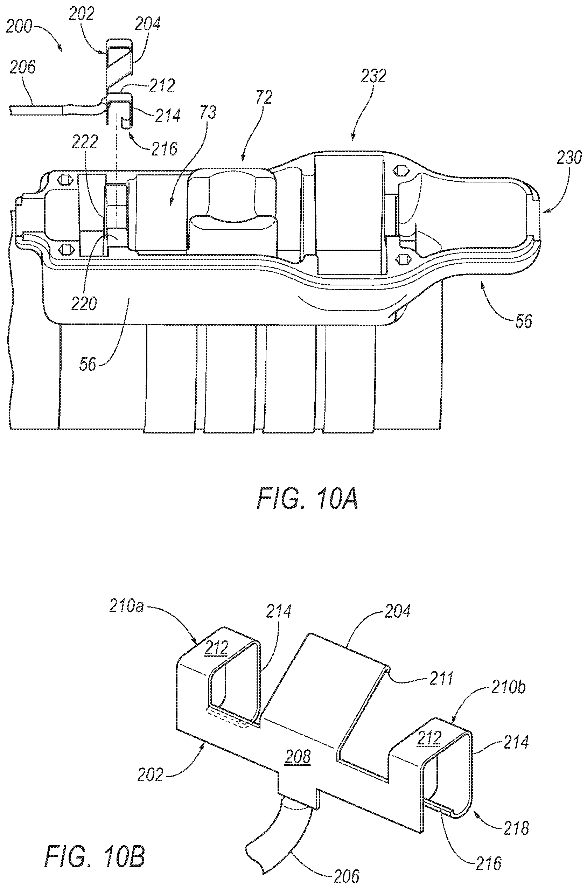

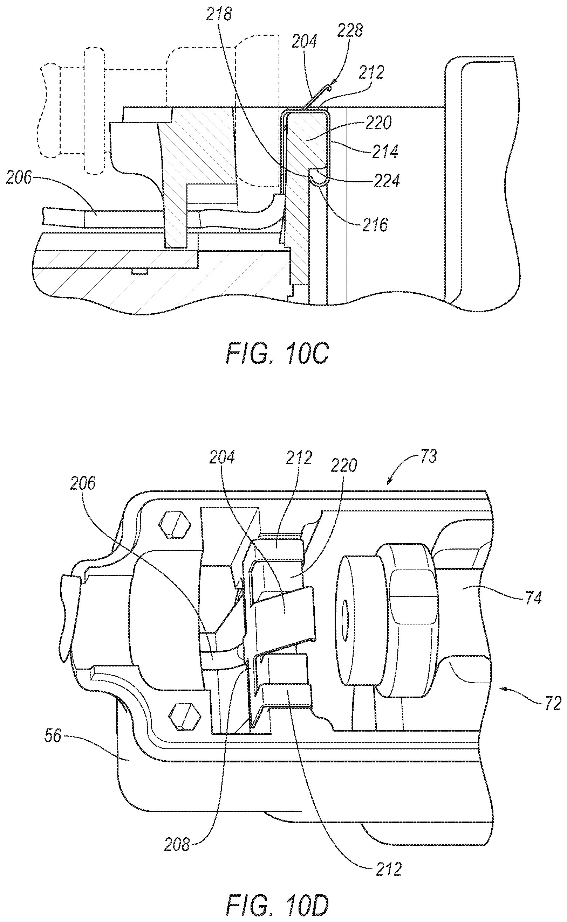

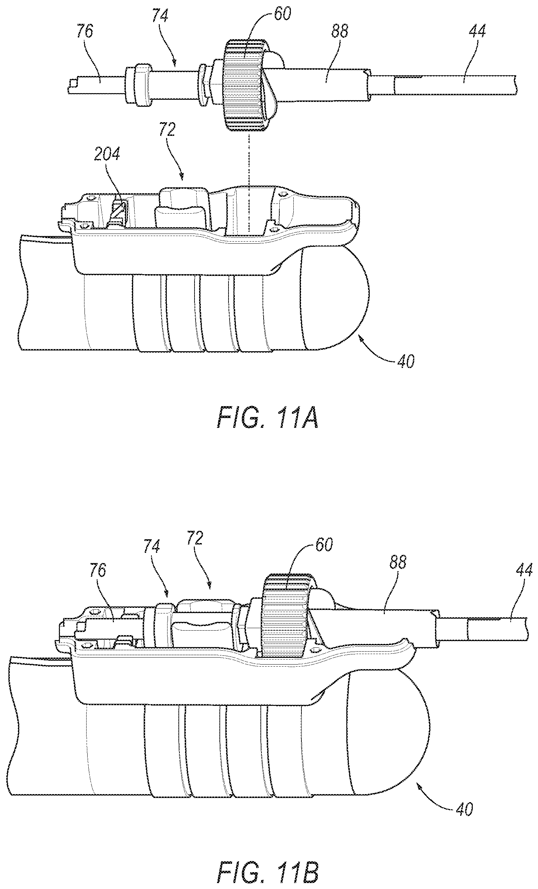

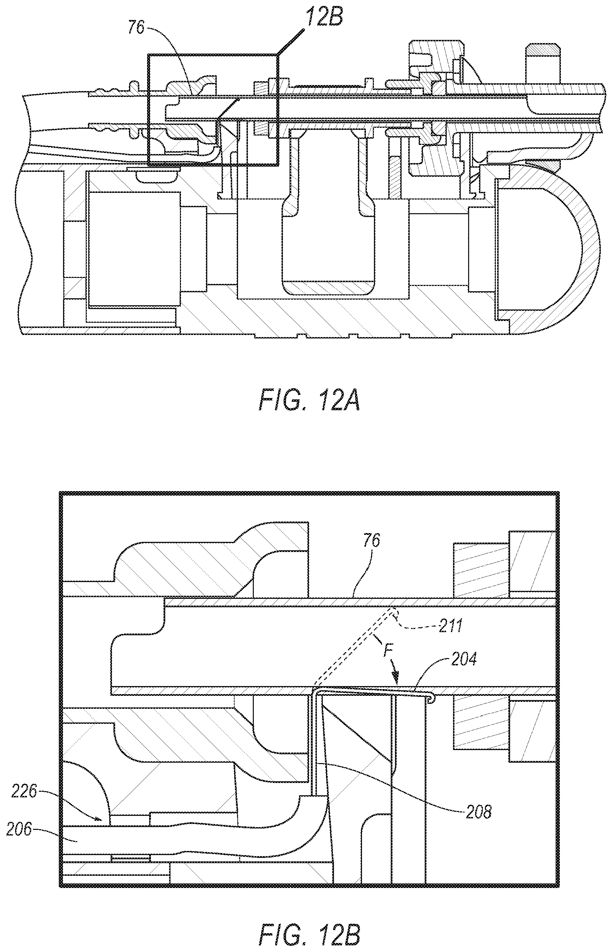

Referring now to FIGS. 10-12C, a neuro stimulator adapter assembly 200 for use with a tissue removal device, such as tissue removal device 40 will now be described. In the embodiment shown in FIGS. 10-12C, neuro stimulator adapter assembly 200 comprises at least one connector element 202, a contact element 204 and a communicating wire 206. The connector element 202 may comprise a main body section 208 and a pair of clip members 210a, 210b. The main body section 208 has the contact element 204 hingedly attached thereto so as to be spring loaded. The communicating wire 206 is operatively connected to the main body section 208. In one exemplary arrangement, the communicating wire 206 is in electrical communication to the main body section 208.

In one exemplary arrangement, the clip members 210a, 210b extend upwardly from the main body section 208 and includes an engagement section 212 that is oriented generally perpendicular to the main body section 208. A gripping section 214 extends downwardly from the engagement section 212, so as to be generally parallel to the main body section 208. The gripping section 212 may further be constructed with a hook member 216 at an end portion 218 of the gripping section 212.

Referring to FIGS. 10C-FIG. 11A, in one exemplary arrangement, the neuro stimulator adapter assembly 200 is mounted within the bottom shell 56 of the upper housing 52. More specifically, the clip members 210a, 210b are mounted over a mounting rail 220 (see FIG. 10A) disposed within the bottom shell 56. The engagement sections 212 are disposed on the mounting rail 220 and the hook members 216 engage around the mounting rail 220 and engage a bottom edge 224 on the mounting rail 220 (FIG. 10C). A distal end of the communication wire 206 extends out of a proximal opening 226 (best seen in FIG. 12B) of the bottom shell 56 such that the communication wire 206 may be electrically connected to a stimulation generator/monitoring console, such as, for example, one manufactured by Medtronic.

Mounting rail 220 further includes a cut-out section 222. The contact element 204 is disposed over the cut-out section 222. While the contact element 204 is hingedly connected to the body portion 208, the contact element 204 is biased in an upwardly extending configuration, as illustrated in FIG. 10C, for example. More specifically, the contact element 204 is biased so as to be disposed upward, at an angle, with a distal end 228 is disposed toward a distal end 230 of the bottom shell 56. In one exemplary arrangement, the contact element 204 is biased at an angle of less than 90° with respect to the engagement sections 212. The contact element 204 may further include a grip element 211 disposed on the free end of contact element 204.

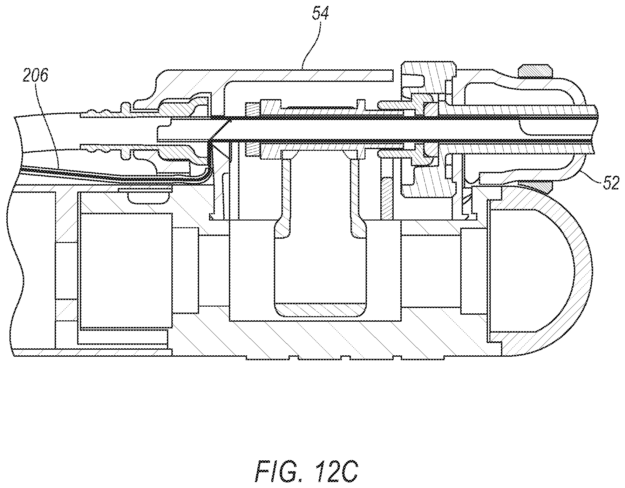

Once the neuro stimulator adapter assembly 200 is mounted to the bottom shell 56, the inner and outer cannula assembly is disposed within the bottom shell 56. More specifically, the rotation dial 60 is positioned within a mounting channel 232 of the bottom shell 56. The cam transfer 74 is disposed on to the cam transfer 72 that extends upwardly into the bottom shell 56. Once the inner and outer cannula assembly is properly positioned, the inner cannula 76 will come into contact with and force the contact element 204 downward as indicated by arrow F in FIG. 12B. The grip element 211, if provided, will frictionally engage an outer edge of the mounting rail 220. The inner and outer cannula assembly is then held in place by the upper shell 54. In this manner, as the inner cannula 76 reciprocates during the cutting operation, contact is maintained between the inner cannula 76 and the contact element 204. Thus, the inner cannula 76 becomes energized via the contact element 204. The electrical signal is then transmitted through the outer cannula 44 toward the distal end 47 via the inner cannula 76 to tissue to stimulate the tissue at an area of interest. Thus, prior to or during a surgical operation, and without the need for a separate monopolar stimulation instrument, electrical signals from the tissue that comes into contact with the outer cannula 44 (or inner cannula 76) may be monitored, in real time, to provide information concerning critical neural structures within an area of interest to assist the clinician in avoiding damage to those neural structures while conducting a cutting operation. Referring to FIG. 12C, the bolded lines indicate the electrical signal transmitted through the inner cannula 76.

Another alternative assembly option is that the upper shell 54 placed over the bottom shell 52 with the neuro stimulation adapter 200 disposed therein. In this manner, the upper shell 54 compressibly retains 200 as the lower shell 52 does with respect to the other components located within the lower shell (such as the inner cannula and outer cannula assembly.) In this arrangement, the hook members 216 may be eliminated.

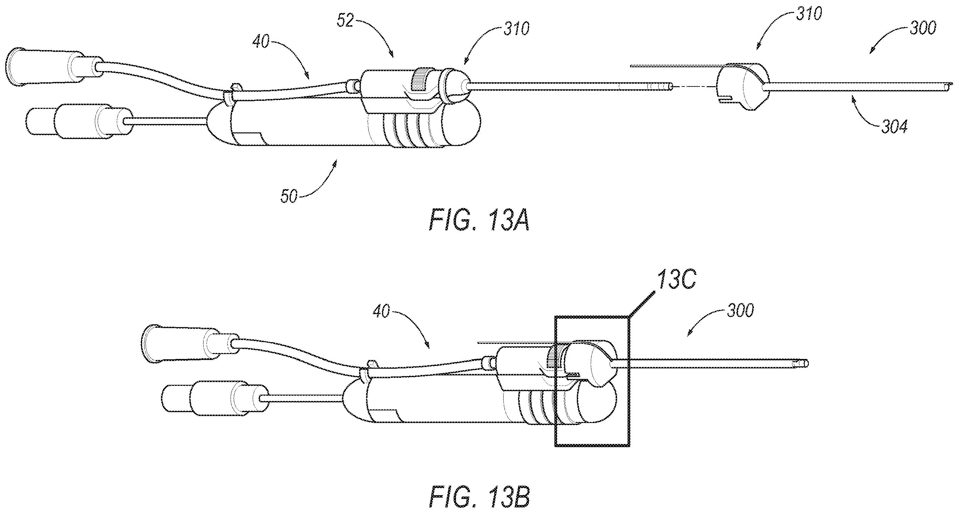

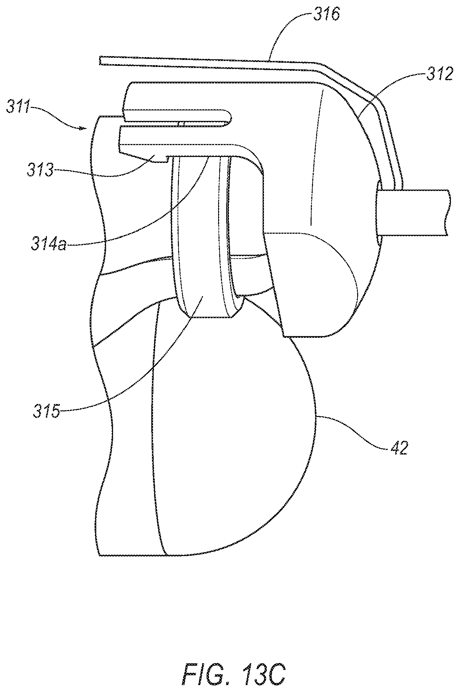

An alternative embodiment of a neuro stimulator arrangement 300 that may be selectively secured to a tissue removal device, such as tissue removal device 40 is shown in FIGs. 13-15. Neuro stimulator arrangement 300 includes a connecting hub 302, a communication cannula 304 and a communicating member 306.

The connecting hub 302 generally includes a cylindrical or semi-cylindrical body 308 that is configured to selectively attach to a portion of a tissue removal device. In one exemplary arrangement, cylindrical body 308 is configured to attach to an upper housing 52 of tissue removal device 40. For example, as depicted in FIGS. 13A-13B, the body 308 of the connecting hub 302 may be configured to connect to the upper housing 52 about a distal end 310 thereof. In one exemplary arrangement, the connecting hub 302 may form an end cap configured to cover the distal end 310 of the upper housing 52 when the hub 302 is in an installed position. In one exemplary arrangement, the hub 302 may be configured to "snap" on to the upper housing 52. When installed, as shown in FIG. 13B, the cylindrical body 308 may extend proximally from the end cap 312 and cover at least a portion of the distal end 310 such that the cylindrical body 308 may extend around approximately 50% of the distal end 310, leaving a portion of the distal end 310 exposed, as shown in FIG. 13C, for example.

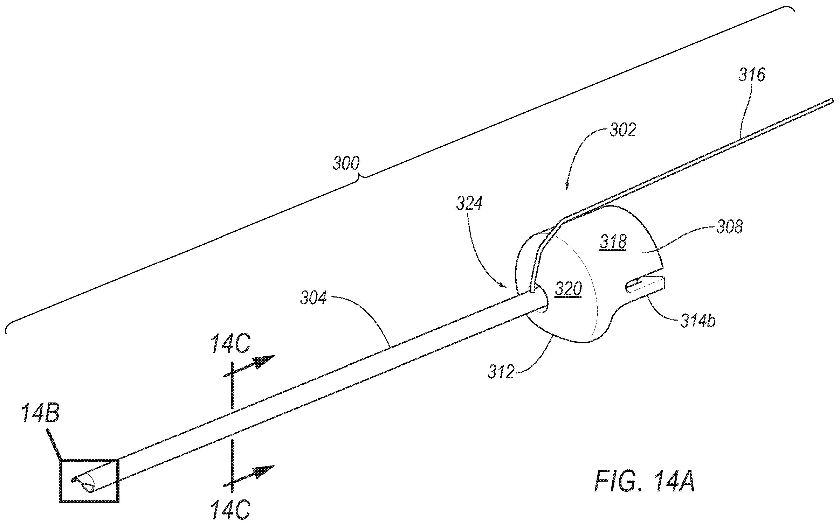

In one exemplary configuration, a proximal end 311 of the hub 302 may include engagement members 313 that snap-fit over a mounting ring 315 disposed around a portion of a tissue removal device, such as the upper housing 52 of the tissue removal device 40 (best seen in FIG. 13C). The mounting ring 315 may be fixedly secured to a portion of the tissue removal device in any known manner, including welding or adhesive. In one exemplary arrangement, once connected, the hub 302 may be selectively rotatable about the distal end 310, without the hub 302 abutting the handpiece 42. An edge 314 of each end of the cylindrical body 308 may abut the handpiece 42 to limit the rotational extent of the cylindrical hub 302. More specifically, the hub 302 may rotate between a first position and a second position where a first edge (e.g., the edge 314a of FIG. 13C) abuts the handpiece 42 and a second position where a second edge (e.g., the edge 314b in FIG. 14A, opposite the edge 314a of FIG. 13C) abut the handpiece 42. The exposed portion defined between the edges 314 defines the degree of rotation of the hub 302. The larger the exposed portion and the smaller the cylindrical body 308, the larger the degree of rotation of the hub 302. In one example, the hub 302 may have up to 200-250 degrees of rotation. Alternatively, the hub 302 may be configured to be fixed at a predetermined location with respect to the upper housing 52.

The hub 302 may further include a guide portion (not shown in connection with the arrangement in FIGS. 13-15, but shown in exemplary arrangements discussed below), that has a guide track configured to receive and maintain an orientation of a communication wire 316. An exemplary guide portion and guide track is shown and described in co-pending U.S. application serial no. 15/348,575. An exemplary guide portion and guide track may include a section on an outer periphery 318 of the cylindrical body 308, and extending from the outer periphery and partially along an end face 320 of the end cap 312 to direct and secure the communication wire 316 to the communication cannula 304.

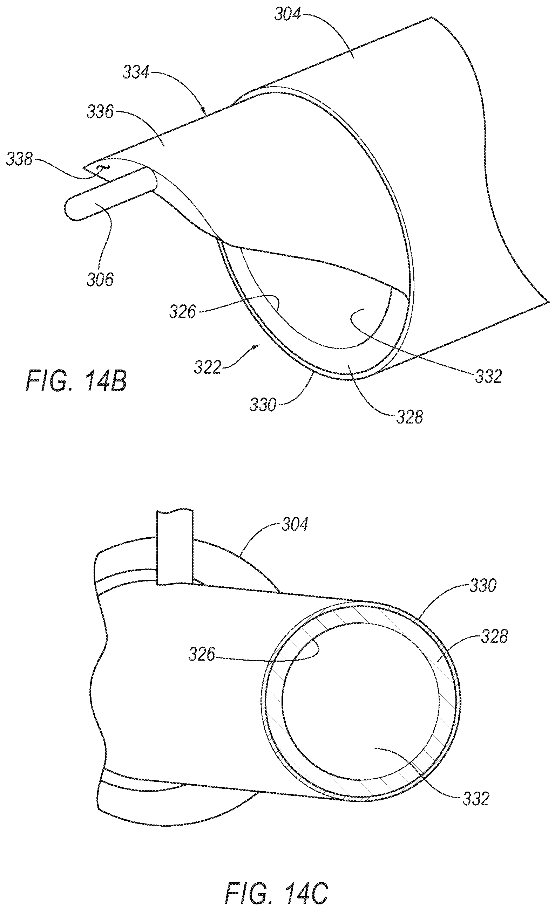

Referring to FIGS. 14A-14C, the communication cannula 304 is defined by a distal end 322 and a proximal end 324. The proximal end 324 is fixedly secured to the hub 302 in any known manner. As best seen in FIG. 14C, the communication cannula 304 comprises multiple layers, an inner layer 326, a communicating layer 328 and an outer layer 330.

The inner layer 326 is an insulating layer defining a lumen 332 therein. The communicating wire 316 is electrically connected to the communicating layer 328. In one exemplary arrangement, a distal end of the communicating wire 316 is welded and sealed to the communicating layer 328. A distal end 334 of the communicating layer 328 may further include an extended portion 336 that extends distally from the inner layer 326 and the outer layer 330. To facilitate ease of placement during use, a portion of the distal end 334 of the communicating layer 328 may be partially cut away, as illustrated in FIG. 14B. However, it is understood that other configurations for the distal end 334 are contemplated. The communication member 306 extends from a distal face 338 of the communication layer 328. In one exemplary arrangement the communication tip 306 is welded or otherwise fixedly secured to the distal face 338. The outer layer 330 is also an insulating layer such that the communicating layer 325 is sandwiched between insulating layers 326, 330. In one exemplary arrangement, a heat shrink material over the communicating layer 328 serves as the outer layer 330.

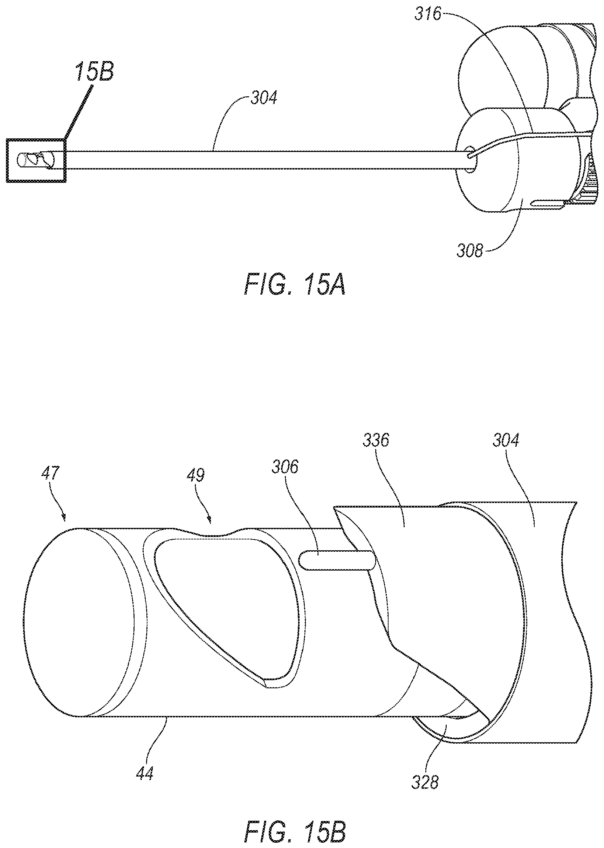

Referring to FIGS. 13A-13C and 15B, in operation, the neuro stimulator arrangement 300 may be selectively slid over a distal end 47 of an outer cannula 44 of tissue removal device 40. In one exemplary arrangement, the proximal end of communication wire 316 is electrically connected to a stimulation/monitoring console (not shown) in a conventional manner. In one exemplary arrangement, the hub 302 may include a guide groove (not shown) formed in the outer surface of the hub 302 in which that communication wire 316 may be disposed. The hub 302 may be selectively rotated about the outer cannula 44 until the communication tip 306 generally aligns with a proximal edge of distal opening 49 of the outer cannula 44, as shown in FIG. 15B. In this manner, the communication tip 306 can be utilized to provide neuro stimulation at tissue adjacent the distal opening 49, which allow for neuro monitoring during a tissue removal operation. Such monitoring may provide real-time monitoring of critical neurological functions and vessels prior to initiating any cutting operation.

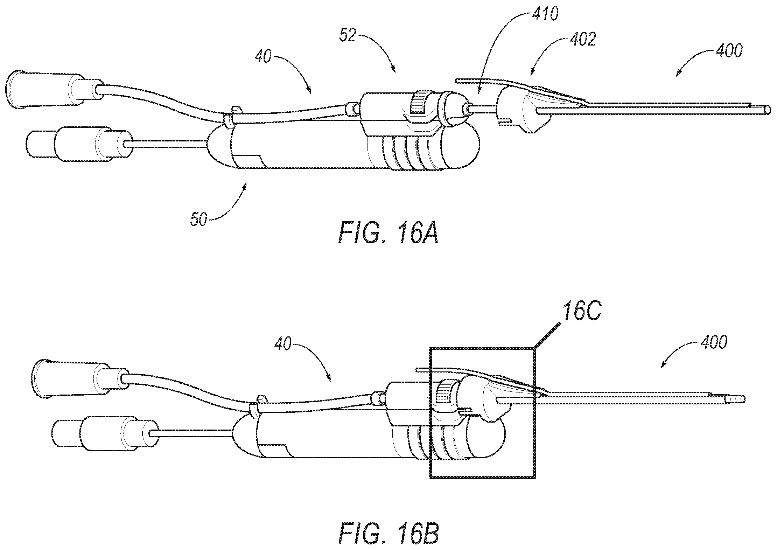

Referring to FIGS. 16A-19C, another alternative neuro stimulation arrangement 400 is illustrated. Neuro stimulator arrangement 400 includes a connecting hub 402, a delivery sleeve 404, an engagement sleeve 405 and a communicating member 406.

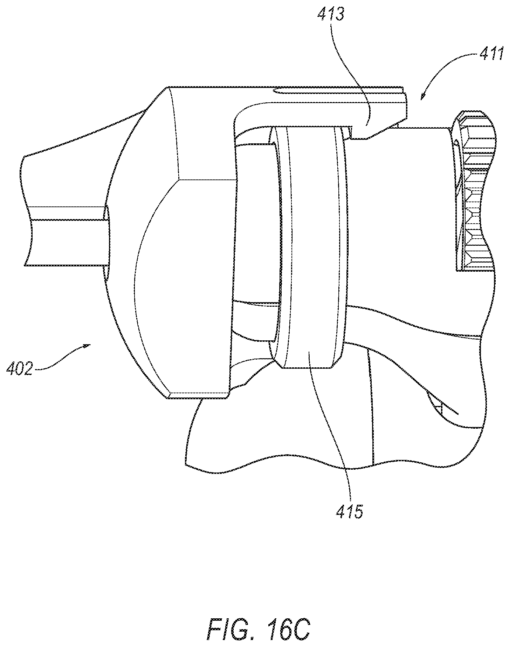

The connecting hub 402 generally includes a cylindrical or semi-cylindrical body 408 that is configured to selectively attach to a portion of a tissue removal device, such as the upper housing 52 of the tissue removal device 40. For example, as depicted in FIGS. 16A-16C, the body 408 of the connecting hub 402 may be configured to connect to the upper housing 52 about a distal end 410 thereof. In one exemplary arrangement, the connecting hub 402 may form an end cap configured to cover the distal end 410 of the upper housing 52 when the hub 402 is in an installed position. In one exemplary arrangement, the hub 402 may be configured to "snap" on to the upper housing 52, as will be explained below. When installed, as shown in FIG. 16B, the cylindrical body 408 may extend from the end cap 412 and cover at least a portion of the distal end 410 such that the cylindrical body 408 may extend around approximately 50% of the distal end 410, leaving a portion of the distal end 410 exposed, as shown in FIG.16C, for example.

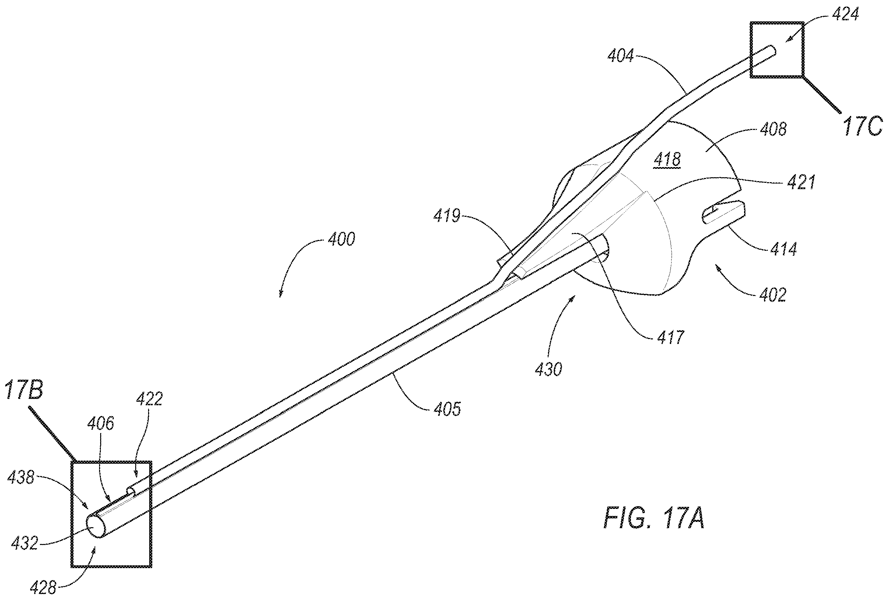

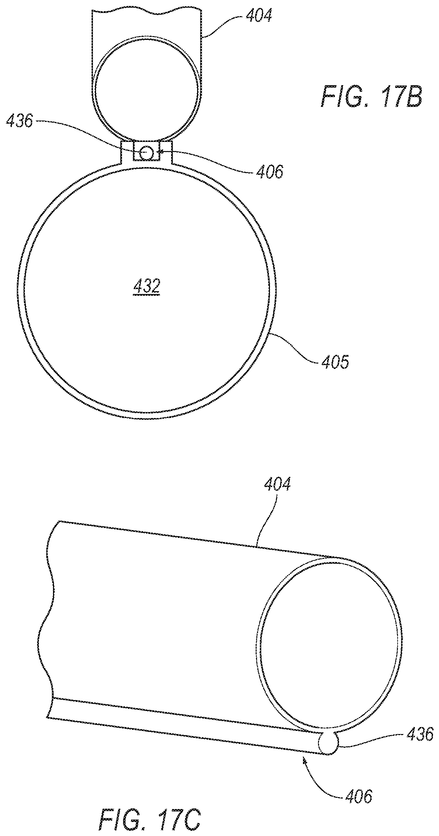

Similar to the arrangement shown in FIGS. 13A-15B, in one exemplary configuration, a proximal end 411 of the hub 302 may include engagement members 413 that may snap-fit over a mounting ring 415 disposed around the upper housing 52 of the tissue removal device 40 (best seen in FIG. 16C). The mounting ring 415 may be fixedly secured to the upper housing 52 or to other components of a tissue removal device in any known manner, including welding or adhesive. Once connected, the hub 402 may be selectively rotatable about the distal end 410, without the hub 402 abutting the handpiece 42. An edge 414 of each end of the cylindrical body 408 may abut the handpiece 42 to limit the rotational extent of the cylindrical hub 402. More specifically, the hub 402 may rotate between a first position and a second position where a first edge (e.g., the edge 414 of FIG. 17A) abuts the handpiece 42 and a second position where a second edge (e.g., the edge opposite the edge 414 of FIG. 13C) abut the handpiece 42. The exposed portion defined between the edges 414 defines the degree of rotation of the hub 402.

The hub 402 may further include a guide portion 417 that defines a guide track 419 configured to receive and direct an orientation of the delivery sleeve 404. In one exemplary configuration, guide portion 417 extends distally from a distal edge 421 of body 408 and slopes toward an outer periphery of engagement sleeve 405, as best shown in FIG. 17A Guide portion 417 is fixedly secured to the body 408 in any known manner. While not shown, it is understood that guide portion 417 may also include a section on an outer periphery 418 of the cylindrical body 408.

The delivery sleeve 404 is defined by a distal end 422 and a proximal end 424. The proximal end 424 extends proximally from the hub 424. In one exemplary arrangement, a distal end 422 of delivery sleeve 404 is positioned proximally from a distal end 428 of the engagement sleeve 405. In one exemplary arrangement, the delivery sleeve 404 may be a flexible sleeve.

The engagement sleeve 405 is defined by the distal end 428 and a proximal end 430 and defines a lumen 432 therebetween. In one exemplary arrangement, the proximal end 430 is fixedly secured to the hub 402.

The communicating member 406 may be constructed as a neurostimulation wire or probe. In one exemplary arrangement, the communicating member 406 is sandwiched between the delivery sleeve 404 and the engagement sleeve 405. The communicating member 406 may be fixedly secured to both the delivery sleeve 404 and the engagement sleeve 405. In one exemplary arrangement, the communicating member 406 may be co-extruded with delivery sleeve 404 and the engagement sleeve 405.

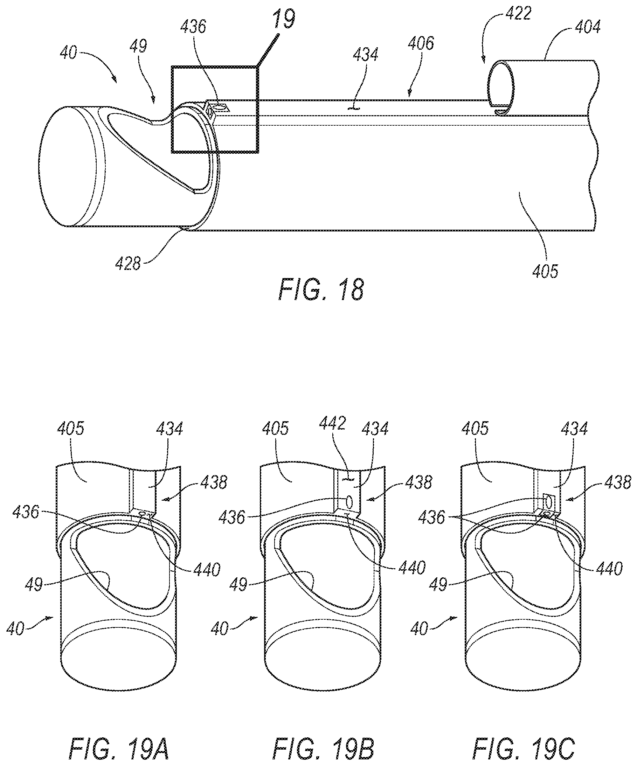

As may be seen in FIG. 18, in one exemplary arrangement, the communicating member 406 extends beyond the distal end 422 of the delivery sleeve 404 to the distal end 428 of the engagement sleeve 405. The communicating member 406 includes an insulation layer 434 and a conductive area 436. In one exemplary arrangement, the insulation layer 434 surrounds a majority of the communicating member 406, with only the conductive area 436 being exposed at a distal end 438 of the communicating member 406. A connecting wire (not shown) may have one end welded adjacent the proximal end of the communicating member 406 and a second end connected to a power source. To achieve the exposure, the distal end 438 of the communicating member 406 may be skived in a variety of configurations to expose the conductive area 436 to create a contact for neuro stimulation and monitoring. Referring to FIGS. 19-19C, a variety of alternative skiving arrangements are shown.

For example, referring to FIG. 19A, in a first exemplary arrangement, a distal end face 440 is skived to expose the conductive area 436. Alternatively, referring to FIG. 19B, a small area is skived on an upper surface 442, to limit the exposure of the conductive area 436 on only the upper surface 442. As yet another exemplary arrangement, referring to FIG. 19C, areas on the upper surface 442 and the end face 440 is skived, to provide exposure of the conductive area 436.

Referring to FIGS. 16A-16C and 18, in operation, the engagement sleeve 405 is slid over the outer cannula 44 of the tissue removal device 40, with the hub 402 engaging a distal end of the housing 410 of the tissue removal device 40. In one exemplary arrangement, the hub 402 is snapped onto the tissue removal device 40 to fixedly secure the hub 402 to the tissue removal device 40. Once connected, a secondary device, such as a fiber optic light (not shown) may be delivered through the delivery sleeve 404. The communicating member 406 may then be electrically connected to a power source or stimulation console. In one exemplary arrangement, the electrical connection for the communication member 406 extends along the delivery sleeve 404, proximal of the hub 402. Alternatively, the communication member 406 extends through the hub 402 to be connected to a power source. As shown best in FIG. 18, once the engagement sleeve 405 is slid over the tissue removal device 40 and the hub 402 is connected, the conductive area 436 is positioned adjacent the distal opening 49 formed in the outer cannula 44. In this manner, instead of running electrical current through the tissue removal device 40 handpiece, a separate attachment provides neuro stimulation close to the opening 47 of the tissue removal device 40. Further, when vacuum is applied to inner cannula lumen 78, the tissue will be drawn toward the opening 47, allowing tissue to be monitored by the communicating member 406 prior to the inner cannula 76 being activated to cut tissue.

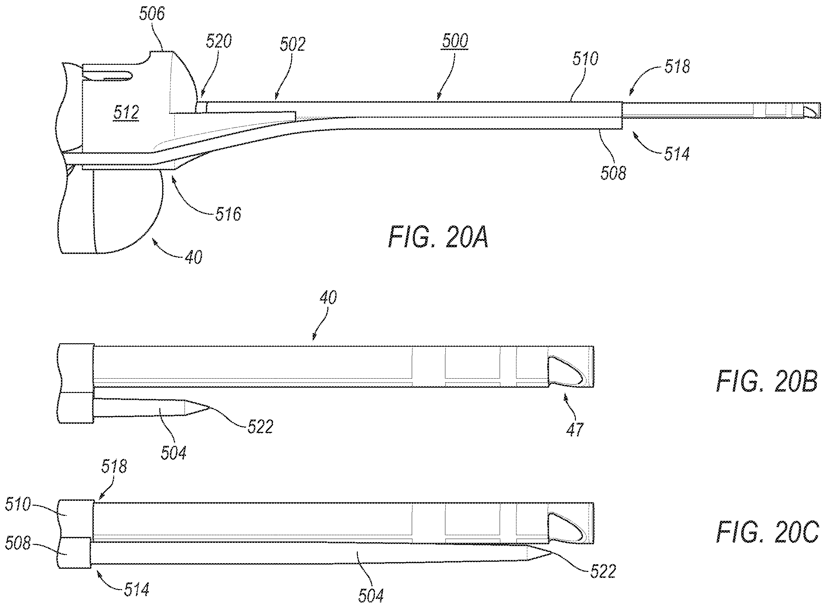

Referring to FIGS. 20A-20C, a further embodiment of an alternative neuro stimulation arrangement 500 is illustrated. Arrangement 500 comprises a delivery sleeve assembly 502 and a neurostimulation probe 504 (best seen in FIGS. 20B-20C). The delivery sleeve assembly 502 is similar to the neuro stimulator arrangement 400 in that the delivery sleeve assembly 502 comprises a connecting hub 506, a delivery sleeve 508 and an engagement sleeve 510.

The connecting hub 506 generally includes a cylindrical or semi-cylindrical body 512 that is configured to selectively attach to the upper housing of the tissue removal device 40. For example, as depicted in FIG. 20A, the body 512 of the connecting hub 506 may be configured to connect to the upper housing about a distal end thereof similar to arrangement 400. In one exemplary arrangement, the hub 506 may be configured to "snap" on to the upper housing 52, similar to the arrangements shown in FIGS. 13A-15B and FIG. 16, or attach via a friction fit. When installed, as shown in FIG. 20A, the cylindrical body 512 may extend from the end cap and cover at least a portion of the distal end such that the cylindrical body 512 may extend around approximately 50% of the distal end, leaving a portion of the distal end exposed.

The delivery sleeve 502 is defined by a distal end 514 and a proximal end 516. The proximal end 516 extends proximally of the hub 506 and may be attached to part of an outer surface of the hub 506. In one exemplary arrangement, the distal end 514 of delivery sleeve 502 may be positioned such that the distal end 514 is in axial alignment with the distal end 518 of the engagement sleeve 510.

The engagement sleeve 510 is defined by the distal end 514 and a proximal end 520 and defines a lumen there between. In one exemplary arrangement, the proximal end 520 is fixedly secured to the hub 506.

In operation, the engagement sleeve 510 is slid over the outer cannula 44 of the tissue removal device 40, with the hub 506 engaging a distal end of the housing of the tissue removal device 40. In one exemplary arrangement, the hub 506 is snapped onto the tissue removal device 40 to fixedly secure the hub 506 to the tissue removal device 40. Once connected, a neurostimulation probe 504 may be advanced through the delivery sleeve 502 until a distal end 522 of the probe 504 extends past the distal end 514 of the delivery sleeve 502, as shown in FIG. 20B. Neurostimulation probe 504 may then be electrically connected to a power source or stimulation console. As shown best in FIG. 20C, once the engagement sleeve 510 is slid over the tissue removal device 40 and the hub 506 is connected, the distal end 522 which is conductive, is positioned adjacent the distal opening 49 formed in the outer cannula 44. In this manner, instead of running electrical current through the tissue removal device 40 handpiece, a separate attachment provides neuro stimulation close to the opening 47 of the tissue removal device 40. Further, when vacuum is applied to inner cannula lumen 78, the tissue will be drawn toward the opening 47, allowing tissue to be monitored by the neurostimulation 504 prior to the inner cannula 76 being activated to cut tissue.

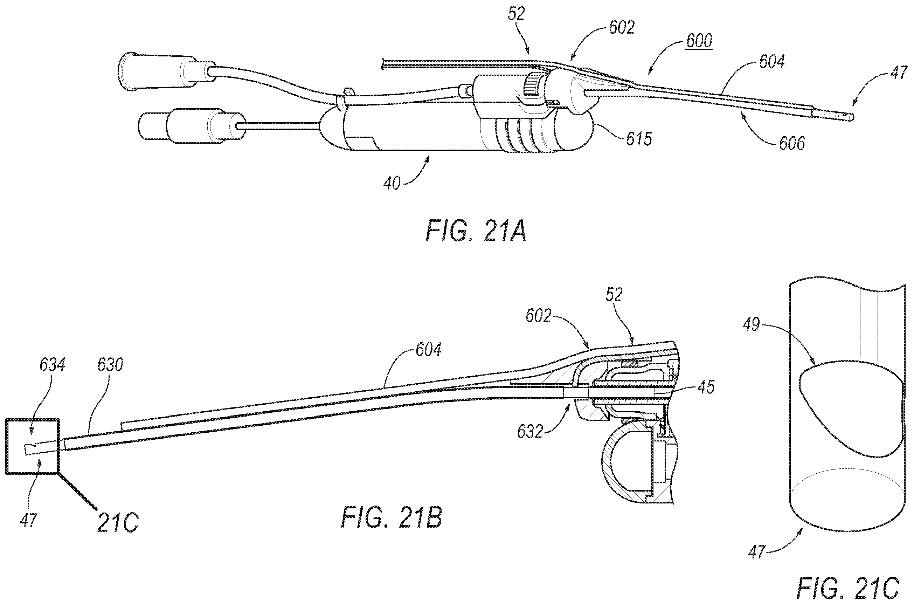

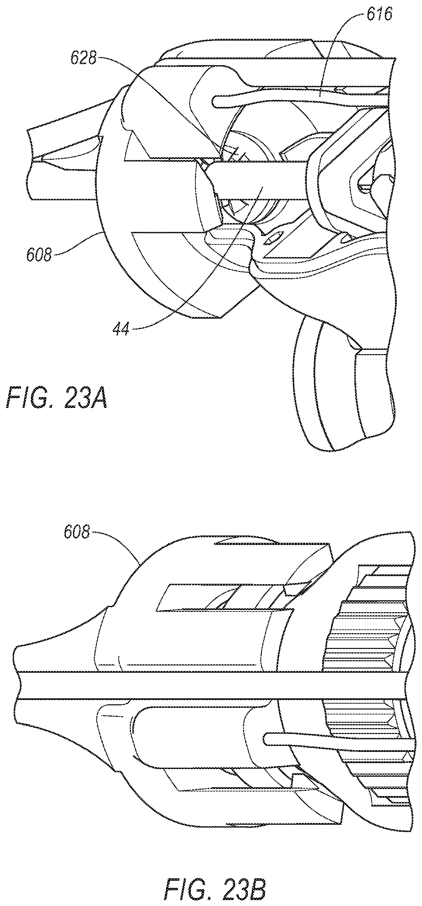

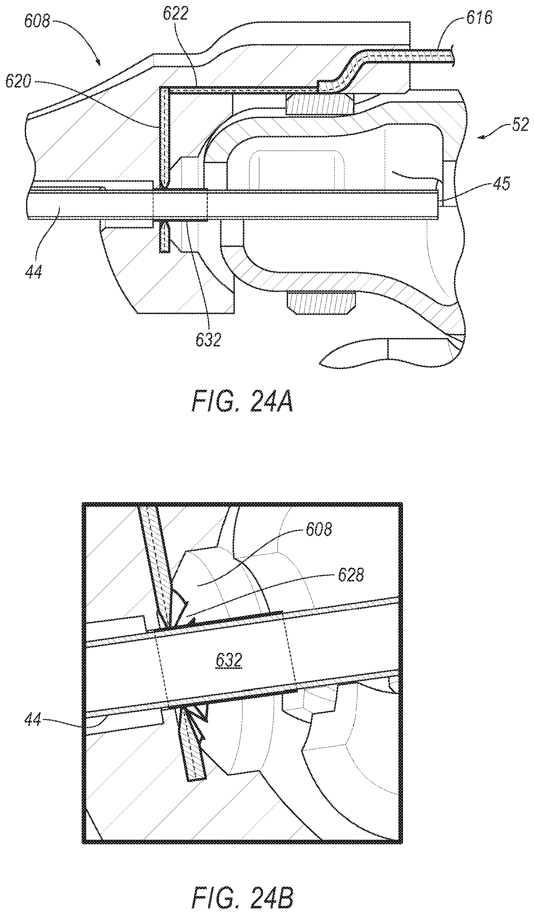

Referring to FIGS. 21A-24B, a further exemplary neuro stimulation arrangement 600 is illustrated. Neuro stimulator arrangement 600 includes a connecting hub 602, a delivery sleeve 604 and an engagement sleeve 606.

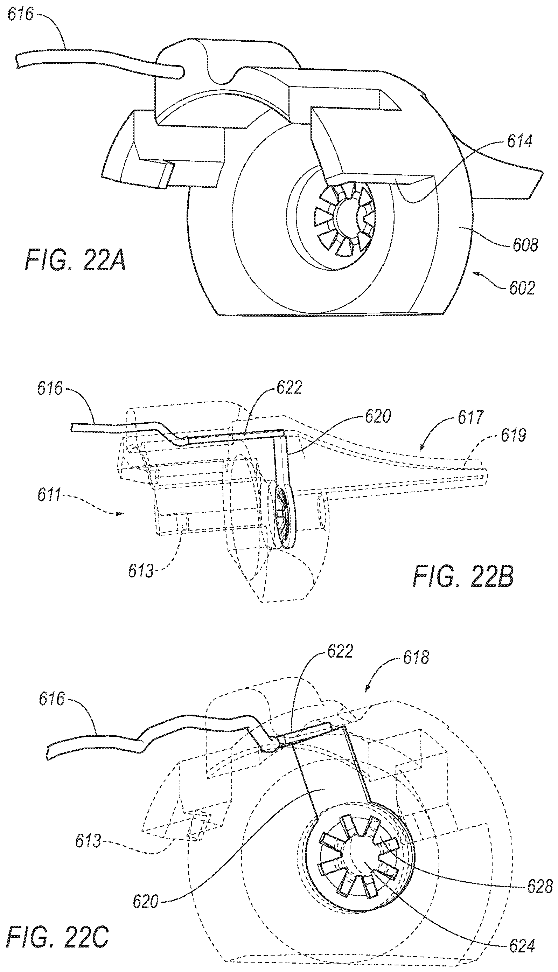

Referring to FIGS. 22a-24b, details of the connecting hub 602 will be described. Connecting hub 602 generally includes at least a semi-cylindrical body 608 that is configured to selectively attach to a portion of a tissue removal device such as tissue removal device 40. In one exemplary arrangement, the body 608 is configured to attach to an upper housing 52 of a tissue removal device. For example, as depicted in FIG. 21A, the body 608 of the connecting hub 602 may be configured to connect to the upper housing 52 about a distal end thereof. In one exemplary arrangement, the connecting hub 602 may form an end cap configured to cover the distal end of the upper housing 52 when the hub 602 is in an installed position. In one exemplary arrangement, the hub 602 may be configured to "snap" on to the upper housing 52, as explained above in connection with other embodiments. When installed, as shown in FIG. 21A, the cylindrical body 608 may extend from the end cap and cover at least a portion of the distal end such that the cylindrical body 608 may extend around approximately 50% of the distal end, leaving a portion of the distal end exposed.

Similar to the arrangement shown in FIGS. 13A-15B, in one exemplary configuration, a proximal end 611 of the hub 602 may include engagement members 613 that snap-fit over a mounting ring 615 disposed around the upper housing 52 of the tissue removal device 40 (best seen in FIG. 21A). The mounting ring 615 may be fixedly secured to the upper housing 52 in any known manner, including welding or adhesive. Alternatively other means of selectively securing the hub 602 to the upper housing 52. In one exemplary arrangement, once connected, the hub 602 may be selectively rotatable about the distal end, without the hub 602 abutting the handpiece 42. An edge 614 of each end of the cylindrical body 608 may abut the handpiece 42 to limit the rotational extent of the cylindrical hub 602. More specifically, the hub 602 may rotate between a first position and a second position where a first edge (e.g., the edge 614 of FIG. 22A) abuts the handpiece 42 and a second position where a second edge (e.g., the edge opposite the edge 614) abuts the handpiece 42. The exposed portion defined between the edges 614 defines the degree of rotation of the hub 602.

The hub 602 may further include a guide portion 617 that defines a guide track 619 configured to receive and direct an orientation of the delivery sleeve 604 similar to that shown in the arrangement 400.

The hub 602 further includes a contact assembly 618, set seen in FIG. 22A-22C. Contact assembly 618 further comprises a contact plate 620, a connector member 622 extending from the contact plate 620 and an engagement aperture 624 extending though the contact plate 620. Surrounding the engagement aperture 624 is at least one contact 628. In one exemplary arrangement, a plurality of contacts 628 are provided with the contacts 628 arranged equidistance about engagement aperture 624. A communication wire 616 operatively connects the contact assembly 618 to a Neuro monitoring/stimulation console (not shown). In one exemplary arrangement, the contact assembly 618 may be insert molded into the body 608 of the hub 602.

In a first exemplary arrangement, during manufacturing, a distal end 47 of outer cannula 44 is closed off and at least a portion of outside surface 630 of the outer cannula 44 is provided with a coating or mask that limits connectivity. In one exemplary arrangement, the coating and/or mask is a parylene coating. The interior wall of the outer cannula that forms outer cannula lumen 110 is uncoated to provide electrical connectively within the inside surface of the outer cannula 44, as will be described below.

Referring to FIG. 21B, in one exemplary arrangement, the coating is provided along the majority of the outside surface 630 of the outer cannula 44. However, a small proximal section 632 and small distal section 634 are left uncoated. In one exemplary arrangement, the proximal section 632 is positioned distally from the proximal end 45 of the outer cannula 44. The distal end section 634 extends from the distal end 47 of the outer cannula 44 proximally past the distal opening 49.

Referring to FIG. 21B, the outer cannula 44 is inserted into the upper housing 52 and fixedly secured thereto. The proximal section 632 is positioned through the engagement aperture 624 of the contact plate 620 such that the contacts 628 are in contact with the outer cannula 44. In operation, when the Neuro monitoring/stimulation console is activated, the communication wire 616 delivers energy to the contact plate 620, thereby energizing the proximal section 632 of the outer cannula 44. The coating/mask insulates the outer shaft 44 along the majority of the outer surface 630 of the outer shaft. However, the distal end section 634, which is uncoated and/or unmasked is exposed. As may be seen in FIG. 21C, this results in the distal end section surrounding the distal opening 49 becoming energized. Accordingly, in this manner, neuro stimulation is provided about the opening 47 of the tissue removal device 40. Further, when vacuum is applied to inner cannula lumen 78, the tissue will be drawn toward the opening 47, allowing tissue to be monitored by neurostimulation prior to the inner cannula 76 being activated to cut or aspirate tissue. In this manner, unlike RF and bipolar devices which may result in noise to a neuro monitoring device, tissue removal can be done in real-time with nerve monitoring in a single device. This is unlike prior art devices which require a clinician to stop, insert a neuro monitoring or stimulation device to resection area, remove the neuro monitoring or stimulation device and reinsert a tissue removal device.

Referring to FIG. 21C, in another exemplary arrangement, during manufacturing, the coating may be disposed along the entirety of the outer surface 630 of the outer cannula 44. The proximal section 632 may be exposed by grinding off the coating. The distal opening 47 may then be created. By creating the distal opening 47, after the coating is applied, the edge 636 surrounding the distal opening 47 will be exposed and will transmit stimulation from the Neuro monitoring/stimulation console.

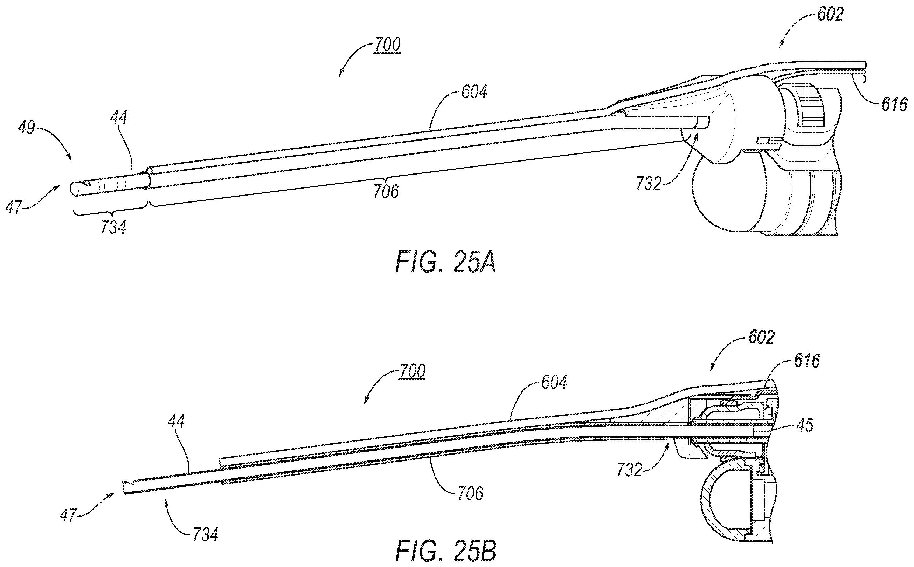

Referring to FIGS. 25A and 25B, an alternative arrangement of an exemplary neuro stimulation arrangement 700 will be discussed. Neuro stimulation arrangement 700 is generally identical to that shown in FIGS. 21A-24B in that it includes the same connecting hub 602, a delivery sleeve 604 and an engagement sleeve 706. Instead of utilizing a coating, insulation of the outer cannula 44 may be accomplished by the engagement sleeve 706. In the embodiment depicted, the engagement sleeve 706 is constructed as an extrusion using a high performance thermoplastic elastomer such as a polyether block amide (PEBA) material. Examples of such PEBA material include PEBEX^{®} and VESTAMID^{®}.

With this arrangement, the majority of the outer cannula 44 is insulated by the engagement sleeve 706. The proximal end section 732 is in engagement with the contacts 628 to deliver stimulation to the distal end section 734, which is exposed. With this arrangement, the neuro stimulation arrangement 700 may be used with any suction or tissue cutting device or any combination of suction and cutting device to convert the device into an alternating tissue removal device and neuro stimulator or simultaneous tissue removal device and neuro stimulator.

In the above exemplary arrangements, the outer cannula 44 can be electrically connected to nerve monitoring instrumentation and the outer cannula may be used as a nerve stimulator to allow for the constant or intermittent stimulation of tissue to determine proximity to and/or to determine the proximity and or continuity of fascicular anatomy or of a larger nerve bundle. With these arrangements, simultaneous monitoring and tissue removal may be performed with a single device.

It will be appreciated that the tissue removal devices and methods described herein have broad applications. The foregoing embodiments were chosen and described in order to illustrate principles of the methods and apparatuses as well as some practical applications. The preceding description enables others skilled in the art to utilize methods and apparatuses in various embodiments and with various modifications as are suited to the particular use contemplated. In accordance with the provisions of the patent statutes, the principles and modes of operation of this invention as claimed, have been explained and illustrated in exemplary embodiments.

It is intended that the scope of the present invention be defined by the following claims. However, it must be understood that this disclosure may be practiced otherwise than is specifically explained and illustrated without departing from its scope. It should be understood by those skilled in the art that various alternatives to the embodiments described herein may be employed in practicing the claims without departing from the scope as defined in the following claims. The scope of the disclosure should be determined, not with reference to the above description, but should instead be determined with reference to the appended claims. It is anticipated and intended that future developments will occur in the arts discussed herein, and that the disclosed systems and methods will be incorporated into such future examples. Furthermore, all terms used in the claims are intended to be given their broadest reasonable constructions and their ordinary meanings as understood by those skilled in the art unless an explicit indication to the contrary is made herein. In particular, use of the singular articles such as "a," "the," "said," etc. should be read to recite one or more of the indicated elements unless a claim recites an explicit limitation to the contrary. It is intended that the following claims define the scope of the invention. In sum, it should be understood that the invention is capable of modification and variation and is limited only by the following claims.

While the above exemplary generally may be categorized as mono-polar arrangements (i.e., where the stimulation element is one of the electrodes and the patient's body is a return electrode to the stimulation/monitoring console), it is understood that many of the above arrangements may be configured to be bi-polar arrangements. For example, in some of the arrangements that utilize a sleeve, one could also provide two electrodes at a fixed distance between two electrode poles such that a signal passes between the poles once touched to a patient's tissue.

## Claims

1. A neurostimulator arrangement comprising:
a tissue removal device (40) comprising
an outer cannula (44) having an open proximal end (45), a closed distal end (47), and a tissue opening (49) proximate the closed distal end (47),
an inner cannula (76) which is partially disposed in a lumen (110) of the outer cannula (44), the inner cannula (76) including a lumen (78) to which vacuum is configured to be applied to aspirate severed tissue samples through the inner cannula (78) in the proximal direction, and
a handpiece (42) into which the proximal end of the outer cannula (44) is connected, wherein the inner cannula (76) is configured to reciprocate within the outer cannula lumen (110) and to cut tissue samples entering the outer cannula (44) via the tissue opening (49); and
a neuro stimulator adapter for use with the tissue removal device (40), the neuro stimulator adapter comprising:
an engagement sleeve (606); and
a hub (602) having a contact assembly (618) disposed therein; the contact assembly (618) configured to be connected to an electrical source, wherein the engagement sleeve (606) is fixedly connected to a portion of hub (602); wherein the contact assembly (618) includes a contact plate (620) having an aperture (624) therethrough, the aperture (624) having at least one contact thereon, wherein the hub (602) may be selectively attached to a portion of the handpiece (42) in an operation mode such that the outer cannula (44) is received within the engagement sleeve (606) and a majority of the outer cannula (44) is insulated by the engagement sleeve (606), with a distal end of the outer cannula (44) protruding distally from the engagement sleeve (606) and a proximal section (632) of the outer cannula (44) being in electrical contact with contact assembly (618), and wherein the vacuum applied to inner cannula lumen (78) is configured to draw tissue toward the tissue opening (47) to allow the tissue to be monitored by a neurostimulation prior to the inner cannula (76) being activated to cut and aspirate the tissue.

2. The neurostimulator arrangement of claim 1, further comprising a communication wire (616) having a first end operatively connected to the contact plate (620), and a second end selectively connectable to a neuro stimulator console.

3. The neurostimulator arrangement of claim 2, wherein a portion of the communication wire (616) is disposed within the hub (602).

4. The neurostimulator arrangement of claim 1, further comprising a connector member (622) that is partially disposed within the hub (602), the connector member (622) fixedly secured to the contact plate (620).

5. The neurostimulator arrangement of claim 4, further comprising a communication wire (616) having a first end fixedly connected to the connector member (622) and a second end selectively connectable to a neuro stimulator module.

6. The neurostimulator arrangement of claim 1, wherein the contact assembly (618) is molded into the hub (602).

7. The neurostimulator arrangement of clam 1, wherein the engagement sleeve (606) is an extrusion formed from a thermoplastic elastomer so as to provide an insulated sleeve.

8. The neurostimulator arrangement of claim 7, wherein the engagement sleeve (606) is constructed from a polyether block amide material.

9. The neurostimulator arrangement of claim 1, further comprising a delivery sleeve (604) partially secured to an outside surface of the engagement sleeve (606).

10. The neurostimulator arrangement of claim 9, wherein a distal end of the delivery sleeve (604) is coplanar with a distal end of the engagement sleeve (606).

11. The neurostimulator arrangement of claim 1, wherein an interior surface of the outer cannula (44) that forms the outer cannula lumen (110) is uncoated to provide electrical connectivity within the inside surface of the outer cannula (44).

## Patentansprüche

1. Neurostimulatoranordnung, umfassend:
eine Gewebeentfernungsvorrichtung (40), umfassend
eine Außenkanüle (44) mit einem offenen proximalen Ende (45), einem geschlossenen distalen Ende (47) und einer Gewebeöffnung (49) nahe dem geschlossenen distalen Ende (47),
eine Innenkanüle (76), die teilweise in einem Lumen (110) der Außenkanüle (44) angeordnet ist, wobei die Innenkanüle (76) ein Lumen (78) umfasst, an das ein Vakuum angelegt werden kann, um abgetrennte Gewebeproben durch die Innenkanüle (78) in proximaler Richtung abzusaugen, und
ein Handstück (42), mit dem das proximale Ende der Außenkanüle (44) verbunden ist, wobei die Innenkanüle (76) dazu eingerichtet ist, sich innerhalb des Lumens (110) der Außenkanüle hin- und herzubewegen und Gewebeproben zu schneiden, die über die Gewebeöffnung (49) in die Außenkanüle (44) eintreten; und
einen Neurostimulatoradapter zur Verwendung mit der Gewebeentfernungsvorrichtung (40), wobei der Neurostimulatoradapter umfasst:
eine Eingriffshülse (606); und
eine Nabe (602), in der eine Kontaktanordnung (618) angeordnet ist; wobei die Kontaktanordnung (618) dazu eingerichtet ist, mit einer elektrischen Quelle verbunden zu werden, wobei die Eingriffshülse (606) fest mit einem Abschnitt der Nabe (602) verbunden ist; wobei die Kontaktanordnung (618) eine Kontaktplatte (620) mit einer sich durch diese erstreckenden Öffnung (624) umfasst, wobei die Öffnung (624) mindestens einen daran angeordneten Kontakt aufweist, wobei die Nabe (602) in einem Betriebsmodus wahlweise an einem Abschnitt des Handstücks (42) befestigt werden kann, sodass die Außenkanüle (44) innerhalb der Eingriffshülse (606) aufgenommen ist und ein überwiegender Teil der Außenkanüle (44) durch die Eingriffshülse (606) isoliert ist, wobei ein distales Ende der Außenkanüle (44) distal aus der Eingriffshülse (606) hervorsteht und ein proximaler Abschnitt (632) der Außenkanüle (44) in elektrischem Kontakt mit der Kontaktanordnung (618) steht, und wobei das an das Lumen (78) der Innenkanüle angelegte Vakuum dazu eingerichtet ist, Gewebe in Richtung der Gewebeöffnung (47) zu ziehen, damit das Gewebe mittels Neurostimulation überwacht werden kann, bevor die Innenkanüle (76) zum Schneiden und Absaugen des Gewebes aktiviert wird.

2. Neurostimulatoranordnung nach Anspruch 1, ferner umfassend eine Kommunikationsleitung (616) mit einem ersten Ende, das betriebsmäßig mit der Kontaktplatte (620) verbunden ist, und einem zweiten Ende, das wahlweise mit einer Neurostimulatorkonsole verbindbar ist.

3. Neurostimulatoranordnung nach Anspruch 2, wobei ein Abschnitt der Kommunikationsleitung (616) innerhalb der Nabe (602) angeordnet ist.

4. Neurostimulatoranordnung nach Anspruch 1, ferner umfassend ein Verbindungselement (622), das teilweise innerhalb der Nabe (602) angeordnet ist, wobei das Verbindungselement (622) fest an der Kontaktplatte (620) befestigt ist.

5. Neurostimulatoranordnung nach Anspruch 4, ferner umfassend eine Kommunikationsleitung (616) mit einem ersten Ende, das fest mit dem Verbindungselement (622) verbunden ist, und einem zweiten Ende, das wahlweise mit einem Neurostimulatormodul verbindbar ist.

6. Neurostimulatoranordnung nach Anspruch 1, wobei die Kontaktanordnung (618) in die Nabe (602) eingeformt ist.

7. Neurostimulatoranordnung nach Anspruch 1, wobei die Eingriffshülse (606) ein aus einem thermoplastischen Elastomer ausgebildetes Extrusionsprofil ist, um eine isolierte Hülse bereitzustellen.

8. Neurostimulatoranordnung nach Anspruch 7, wobei die Eingriffshülse (606) aus einem Polyetherblockamidmaterial hergestellt ist.

9. Neurostimulatoranordnung nach Anspruch 1, ferner umfassend eine Einführhülse (604), die teilweise an einer Außenfläche der Eingriffshülse (606) befestigt ist.

10. Neurostimulatoranordnung nach Anspruch 9, wobei ein distales Ende der Einführhülse (604) mit einem distalen Ende der Eingriffshülse (606) komplanar ist.

11. Neurostimulatoranordnung nach Anspruch 1, wobei eine Innenfläche der Außenkanüle (44), die das Lumen (110) der Außenkanüle bildet, unbeschichtet ist, um eine elektrische Leitfähigkeit entlang der Innenfläche der Außenkanüle (44) bereitzustellen.

## Revendications

1. Agencement de neurostimulation comprenant:
un dispositif d'ablation de tissu (40) comprenant
une canule externe (44) comportant une extrémité proximale ouverte (45), une extrémité distale fermée (47) et une ouverture pour tissu (49) située à proximité de l'extrémité distale fermée (47),
une canule interne (76) qui est , en partie, logée dans une lumière (110) de la canule externe (44), la canule interne (76) comportant une lumière (78) à laquelle peut être appliqué un vide afin d'aspirer des échantillons de tissu sectionnés à travers la canule interne (78) dans la direction proximale, et
une pièce à main (42) à laquelle est raccordée l'extrémité proximale de la canule externe (44), la canule interne (76) étant conçue pour effectuer un mouvement de va-et-vient à l'intérieur de la lumière (110) de la canule externe et pour couper des échantillons de tissu pénétrant dans la canule externe (44) par l'ouverture pour tissu (49); et
un adaptateur de neurostimulateur destiné à être utilisé avec le dispositif d'ablation de tissu (40), l'adaptateur de neurostimulateur comprenant:
un manchon de mise en prise (606); et
un moyeu (602) comportant un ensemble contact (618) disposé en son sein; l'ensemble contact (618) étant conçu pour être connecté à une source électrique, le manchon de mise en prise (606) étant relié à demeure à une partie du moyeu (602); l'ensemble contact (618) comprend une plaque de contact (620) présentant une ouverture (624), l'ouverture (624) comportant au moins un contact, le moyeu (602) pouvant être fixé de manière sélective à une partie de la pièce à main (42) lors du fonctionnement de telle sorte que la canule externe (44) soit logée à l'intérieur du manchon de mise en prise (606) et qu'une majeure partie de la canule externe (44) soit isolée par le manchon de mise en prise (606), une extrémité distale de la canule externe (44) faisant saillie distalement du manchon de mise en prise (606) et une section proximale (632) de la canule externe (44) étant en contact électrique avec l'ensemble contact (618), et le vide appliqué à la lumière de canule interne (78) est configuré pour attirer le tissu vers l'ouverture pour tissu (47) afin de permettre la surveillance du tissu par neurostimulation avant que la canule interne (76) ne soit activée pour couper et aspirer le tissu.

2. Agencement de neurostimulation selon la revendication 1, comprenant en outre un câble de communication (616) dont une première extrémité est reliée de manière fonctionnelle à la plaque de contact (620) et dont une seconde extrémité peut être connectée de manière sélective à une console de neurostimulation.

3. Agencement de neurostimulation selon la revendication 2, dans lequel une partie du câble de communication (616) est logée à l'intérieur du moyeu (602).

4. Agencement de neurostimulation selon la revendication 1, comprenant en outre un élément de connexion (622) qui est , en partie, logé à l'intérieur du moyeu (602), l'élément de connexion (622) étant fixé à demeure à la plaque de contact (620).

5. Agencement de neurostimulation selon la revendication 4, comprenant en outre un câble de communication (616) dont une première extrémité est reliée à demeure à l'élément de connexion (622) et dont une seconde extrémité peut être reliée de manière sélective à un module de neurostimulation.

6. Agencement de neurostimulation selon la revendication 1, dans lequel l'ensemble contact (618) est moulé dans le moyeu (602).

7. Agencement de neurostimulation selon la revendication 1, dans lequel le manchon de mise en prise (606) est un organe formé par extrusion fabriqué à partir d'un élastomère thermoplastique, de manière à former un manchon isolant.

8. Agencement de neurostimulation selon la revendication 7, dans lequel le manchon de mise en prise (606) est fabriqué à partir d'un matériau à base de polyéther-bloc-amide.

9. Agencement de neurostimulation selon la revendication 1, comprenant en outre un manchon de distribution (604) fixé, à certains endroits, à une surface extérieure du manchon de mise en prise (606).

10. Agencement de neurostimulation selon la revendication 9, dans lequel l'extrémité distale du manchon de distribution (604) est coplanaire avec l'extrémité distale du manchon de mise en prise (606).

11. Agencement de neurostimulation selon la revendication 1, dans lequel une surface interne de la canule externe (44), qui forme la lumière de canule externe (110) n'est pas revêtue afin d'assurer une connectivité électrique dans la surface interne de la canule externe (44).
